# EUROPEAN PATENT APPLICATION

(11) **EP 4 737 589 A2**
(43) Date of publication of application: **06.05.2026**
(21) Application number: 26167339.6
(22) Date of filing: 26.05.2022
(51) Int. Cl.: C12Q 1/6869

(54) **OLIGO-MODIFIED NUCLEOTIDE ANALOGUES FOR NUCLEIC ACID PREPARATION**

(30) Priority: 28.05.2021 US 202163194681 P
(62) Divisional of application: 22736057.5
(71) Applicant: Illumina, Inc., San Diego, CA 92122 (US)
(72) Inventor: GORMLEY, Niall Anthony, Cambridge CB21 6DF (GB); RANDISE-HINCHLIFF, Carlo, San Diego, CA 92122 (US); BRODIN, Jeffrey, San Diego, CA 92122 (US); MUSGRAVE-BROWN, Esther, Cambridge CB21 6DF (GB); SHULTZABERGER, Sarah E, San Diego, CA 92122 (US); SLATTER, Andrew, Cambridge CB21 6DF (GB); FISHER, Jeffrey S, San Diego, CA 92122 (US)
(74) Representative: Williams, Andrea

(57) **Abstract**

Nucleic acid techniques are disclosed. Embodiments include modified nucleotides with oligonucleotide adapters that are coupled via cleavable linkers. Incorporation of the modified nucleotide at a 3' end of a nucleic acid permits end-adapterization via ligation of a free 5' end of the oligonucleotide adapter to a 3' reactive group of the modified nucleotide and cleavage at the cleavable linker to liberate a free 3' end.

## Description

### BACKGROUND

The disclosed technology relates generally to techniques for preparing nucleic acids, e.g., sequencing library preparations, using oligo-modified nucleotide analogues. The oligo-modified nucleotide analogues including oligonucleotide adapters can be used to directly incorporate adapters onto nucleic acids as part of sample preparation for downstream processing steps.

The subject matter discussed in this section should not be assumed to be prior art merely as a result of its mention in this section. Similarly, a problem mentioned in this section or associated with the subject matter provided as background should not be assumed to have been previously recognized in the prior art. The subject matter in this section merely represents different approaches, which in and of themselves can also correspond to implementations of the claimed technology.

Molecular biology now makes intensive use of nucleic acid analysis. Various nucleic acid analysis techniques involve a sample preparation stage in which the sample is manipulated to generate an end product that is compatible with the desired analysis platform. For example, certain sequencing platforms are compatible with sequencing libraries that include specific adapter sequences to permit strand capture and synthesis. These adapter sequences may include universal adapters for high throughput parallel processing of large numbers of nucleic acids.

The addition of universal adapters for sequencing can be achieved by a variety of methods. In one example, adapters that contain universal priming sequences can be ligated onto the ends of template nucleic acids. A single adapter or two different adapters may be used in a ligation reaction. If a template nucleic acid has been manipulated such that its ends are the same, i.e., both are blunt or both have the same overhang, then ligation of a single compatible adapter will generate a template with that adapter on both ends. However, if two compatible and different adapters, e.g., adapter A and adapter B, are used, then three permutations of ligated products are formed: template with adapter A on both ends, template with adapter B on both ends, and template with adapter A on one end and adapter B on the other end. This last product is, under some circumstances, the only desired product from the ligation reaction and, consequently, additional purification steps are necessary following the ligation reaction to purify it from the undesired ligation products that have the same adapter at both ends.

Thus, certain techniques for adding universal adapters to a sample involve inherent loss of the sample as well as additional purification steps when creating and subsequently selecting suitably modified fragments. Accordingly, more efficient techniques for adding adapters to nucleic acids are of interest.

### BRIEF DESCRIPTION

In one embodiment, the present disclosure provides an oligo-modified nucleic acid analogue composition. The composition includes a modified nucleotide comprising: a ribose (e.g., a deoxyribose, a ribonucleic acid, a dideoxyribose); a 5' phosphate coupled to the deoxyribose; a 3' reactive group coupled to the ribose; and an oligonucleotide adapter coupled to the ribose by a linker, e.g., a cleavable linker, and terminating in a reactive 5' oligonucleotide end. In an embodiment, the oligonucleotide can be coupled to the linker with a terminating 3' end or a reactive 3' end, depending on desired subsequent reactions after reactions with the modified nucleotide.

In one embodiment, the present disclosure provides a method of modifying a nucleic acid. The method includes providing a nucleic acid and contacting the nucleic acid with a modified nucleotide. The modified nucleotide includes a deoxyribose; a 5' phosphate group coupled to the deoxyribose; a 3' reactive group coupled to the deoxyribose; and an oligonucleotide adapter coupled to the deoxyribose by a cleavable linker and terminating in a 5' oligonucleotide end. The method includes incorporating the modified nucleotide onto a 3' end of the nucleic acid to generate an extended nucleic acid; reacting the 5' oligonucleotide end of the oligonucleotide adapter on the extended nucleic acid with the 3' reactive group to couple the 5' oligonucleotide end to the deoxyribose and such that the oligonucleotide adapter forms a loop. In an embodiment, the method includes cleaving the linker to liberate a 3' end of the oligonucleotide adapter.

In one embodiment, the present disclosure provides a method of modifying a nucleic acid. The method includes contacting a single-stranded nucleic acid with a modified nucleotide comprising: a deoxyribose; a 5' phosphate group coupled to the deoxyribose; and a single-stranded oligonucleotide adapter coupled to the deoxyribose and terminating in a 5' oligonucleotide end. The method also includes using a polymerase to incorporate the modified nucleotide onto a 3' end of the single-stranded nucleic acid to generate an extended single-stranded nucleic acid; annealing a primer comprising a recognition site for a 5' region of the single-stranded oligonucleotide adapter; and extending the primer to synthesize a complementary strand of the single-stranded nucleic acid.

In one embodiment, the present disclosure provides a method of preparing a sequencing library. The method includes providing a double-stranded nucleic acid sample and tagmenting the double-stranded nucleic acid sample using transposome homodimers to incorporate a first adapter on 5' ends of double-stranded fragments generated from the double-stranded nucleic acid sample. The method also includes contacting the double-stranded fragments with modified nucleic acids comprising: a deoxyribose; a 3' reactive group coupled to the deoxyribose; and an oligonucleotide adapter coupled to the deoxyribose by a cleavable linker and terminating in a 5' oligonucleotide end. The method also includes incorporating the modified nucleotides onto 3' ends of the double-stranded fragments to generate an extended nucleic acid; reacting respective 5' oligonucleotide ends of the modified nucleotides with corresponding 3' reactive groups to couple the 5' oligonucleotide ends to the deoxyribose and such that oligonucleotide adapters of the modified nucleotides form loops; and cleaving cleavable linkers of the modified nucleotides liberate 3' ends of the oligonucleotide adapter to generate adapterized double-stranded nucleic fragments of a sequencing library.

In one embodiment, the present disclosure provides a method of preparing a sequencing library. The method includes providing a double-stranded nucleic acid sample. The method also includes contacting the double-stranded fragments with modified adenosines, an individual modified adenosine comprising: a deoxyribose; a 5' phosphate group coupled to the deoxyribose; a 3' reactive group coupled to the deoxyribose; an adenine nucleobase coupled to the deoxyribose; and an oligonucleotide adapter coupled to the deoxyribose or the adenine nucleobase by a linker, wherein the oligonucleotide adapter comprises a forked adapter comprising a first fork, a second fork, and double-stranded portion, the double-stranded portion comprising a 3' thymine overhang, and wherein the linker is coupled to the first fork. The method also includes incorporating the modified adenosines onto 3' ends of the double-stranded nucleic acid via the 5' phosphate group to generate an extended nucleic acid comprising 3' modified adenosine ends; ligating the double-stranded portion of the forked adapter to the 3' modified adenosine ends of the double-stranded nucleic acid via the 3' thymine overhang; and cleaving the linker from the first fork subsequent to the ligating to generate double-stranded nucleic acid having the forked adapter at both ends.

In one embodiment, the present disclosure provides a method of preparing a sequencing library. The method includes providing a double-stranded nucleic acid sample. The method also includes contacting the double-stranded fragments with modified contacting the double-stranded nucleic acid with modified adenosines, an individual modified adenosine comprising: a deoxyribose; a 5' phosphate group coupled to the deoxyribose; a 3' reactive group coupled to the deoxyribose; an adenine nucleobase coupled to the deoxyribose; and a first oligonucleotide adapter coupled to the deoxyribose or the adenine nucleobase by a first linker. The method also includes incorporating the modified adenosines onto 3' ends of the double-stranded nucleic acid via the 5' phosphate group to generate an extended nucleic acid comprising a 3' modified adenosine ends; contacting the extended nucleic acid comprising 3' modified adenosine ends with a forked adapter comprising a first fork, a second fork, a double-stranded portion, the double-stranded portion comprising a 3' thymine overhang, and a second oligonucleotide adapter complementary to the first oligonucleotide adapter, the second oligonucleotide adapter extending from the first fork or the second fork via a second linker, to allow the first oligonucleotide adapter to hybridize to the second oligonucleotide adapter; ligating the double-stranded portion of the forked adapter to the 3' modified adenosine ends of the double-stranded nucleic acid via the 3' thymine overhang; and cleaving the first linker and the second linker to generate double-stranded nucleic acid having the forked adapter at both ends.

In one embodiment, the present disclosure provides a nucleic acid fragment that includes a single or double-stranded nucleic acid fragment; and a modified nucleotide coupled to a 3' end of the nucleic acid fragment, the modified nucleic acid comprising: an oligonucleotide adapter coupled to the ribose by a linker at a first end and terminating in a 5' or 3' oligonucleotide end at a second end.

In one embodiment, the present disclosure provides a method of modifying a nucleic acid. The method includes contacting a double-stranded nucleic acid with an a modified nucleotide comprising: a deoxyribose; a 5' phosphate group coupled to the deoxyribose; and a single-stranded oligonucleotide adapter coupled to the deoxyribose via a linker at a first end and terminating in a 3' oligonucleotide end at a second end. The method also includes incorporating the modified nucleotide onto a 3' end of a first strand of the double-stranded nucleic acid via the 5' phosphate group to generate an extended first strand; annealing a primer comprising a recognition site for a 3' region of the single-stranded oligonucleotide adapter; and extending the primer using a polymerase with 5' to 3' exonuclease activity to synthesize a complementary strand of the single-stranded nucleic acid while degrading a 5' portion of a second strand of the double-stranded nucleic acid.

In one embodiment, the present disclosure provides a method of modifying a nucleic acid. The method includes contacting a double-stranded nucleic acid with an a modified nucleotide comprising: a deoxyribose; a 5' phosphate group coupled to the deoxyribose; and a single-stranded oligonucleotide adapter coupled to the deoxyribose. The method also includes incorporating the modified nucleotide onto a recessed 3' end of a first strand of the double-stranded nucleic acid via the 5' phosphate group to generate an extended first strand; extending the first strand from a 3' end of the modified nucleotide; annealing a single-stranded portion of a forked adapter to the single-stranded oligonucleotide adapter; and ligating a double-stranded portion of the forked adapter to ends of the double-stranded nucleic acid.

In one embodiment, the present disclosure provides a method of modifying a nucleic acid. The method includes contacting a single-stranded RNA with a plurality of single-stranded oligonucleotides comprising a 3' random portion and a 5' fixed sequence portion such that a 3' random portion of one of the plurality of single-stranded oligonucleotides anneals to a 3' end of the single-stranded RNA and such that the 5' fixed sequence portion does not anneal to the single-stranded RNA. The method also includes incorporating a modified nucleotide onto a 3' end of the single-stranded RNA using the fixed sequence portion as a template, wherein the modified nucleotide comprises: a deoxyribose; a 5' phosphate group coupled to the deoxyribose; and a single-stranded oligonucleotide adapter coupled to the deoxyribose and terminating in a free 3' end.

The preceding description is presented to enable the making and use of the technology disclosed. Various modifications to the disclosed implementations will be apparent, and the general principles defined herein may be applied to other implementations and applications without departing from the spirit and scope of the technology disclosed. Thus, the technology disclosed is not intended to be limited to the implementations shown, but is to be accorded the widest scope consistent with the principles and features disclosed herein. The scope of the technology disclosed is defined by the appended claims.

### BRIEF DESCRIPTION OF THE DRAWINGS

These and other features, aspects, and advantages of the present invention will become better understood when the following detailed description is read with reference to the accompanying drawings in which like characters represent like parts throughout the drawings, wherein:
FIG. 1 is a schematic illustration of a modified nucleotide, e.g., an oligo-modified nucleotide analogue, in accordance with embodiments of the present disclosure.
FIG. 2 is a schematic illustration of a process for incorporating a modified nucleotide onto a 3' end of a double-stranded nucleic acid, in accordance with embodiments of the present disclosure.
FIG. 3 is a schematic illustration of a process for incorporating a modified nucleotide including a hybridized adapter onto a 3' end of a double-stranded nucleic acid, in accordance with embodiments of the present disclosure.
FIG. 4 is a schematic illustration of a process for incorporating a modified nucleotide including a forked adapter onto a 3' end of a double-stranded nucleic acid, in accordance with embodiments of the present disclosure.
FIG. 5 is a schematic illustration of a process for incorporating a modified nucleotide onto a 3' end of a double-stranded nucleic acid to enhance a ligation of a forked adapter, in accordance with embodiments of the present disclosure.
FIG. 6A shows an example prior art tagmentation workflow.
FIG. 6B shows an example prior art tagmentation workflow.
FIG. 6C shows an example tagmentation workflows using modified nucleotides, in accordance with embodiments of the present disclosure.
FIG. 7 shows an adapter primer extension across the modified nucleotide, in accordance with embodiments of the present disclosure.
FIG.8 shows a lure-based adapter ligation using a modified nucleotide, in accordance with embodiments of the present disclosure.
FIG. 10 is a schematic illustration of primer extension as part of cDNA synthesis using modified nucleotides, in accordance with embodiments of the present disclosure.
FIG. 11 is a schematic illustration of a process for priming from an oligonucleotide adapter of a modified nucleotide incorporated onto a 3' end of a nucleic acid, in accordance with embodiments of the present disclosure.
FIG. 12 is a schematic illustration of a process of strand extension from an oligonucleotide adapter of a modified nucleotide incorporated onto a 3' end of a nucleic acid, in accordance with embodiments of the present disclosure.
FIG. 13 is a schematic illustration of a process of strand extension from an oligonucleotide adapter loop, in accordance with embodiments of the present disclosure.
FIG. 14 is an illustration of modified nucleotide incorporation onto blunt and recessed duplex DNA and resultant product sizes.
FIG. 15 shows results of modified nucleotide incorporation onto blunt and recessed duplex DNA.
FIG. 16 is an illustration of modified nucleotide primed extension onto blunt and recessed duplex DNA.
FIG. 17 shows results of modified nucleotide primed extension onto blunt and recessed duplex DNA.
FIG. 18 is an illustration of adapter ligation onto blunt duplex DNA including a modified nucleotide.
FIG. 19 shows results of a lure-based adapter ligation.
FIG. 20 shows results of a lure-based adapter ligation.
FIG. 21 shows exonuclease inhibition based on modified nucleotide scar size.

### DETAILED DESCRIPTION

The following discussion is presented to enable any person skilled in the art to make and use the technology disclosed, and is provided in the context of a particular application and its requirements. Various modifications to the disclosed implementations will be readily apparent to those skilled in the art, and the general principles defined herein may be applied to other implementations and applications without departing from the spirit and scope of the technology disclosed. Thus, the technology disclosed is not intended to be limited to the implementations shown, but is to be accorded the widest scope consistent with the principles and features disclosed herein.

The disclosed techniques are directed to oligo-modified nucleotide analogues (e.g., modified nucleotides, as provided herein) and techniques for using the same. The oligo-modified nucleotide analogues harness the ability of polymerases to catalyze the incorporation of nucleotides that are coupled to oligonucleotide adapters (or other functional sequences). Thus, in embodiments, desired sequences can be added to 3' ends of nucleic acids via a polymerase-mediated reaction rather than a ligation reaction, and the use of polymerase permits higher yield of the desired end product relative to ligation. In an embodiment, the disclosed oligo-modified nucleotide analogues include adapter sequences that are used to incorporate adapters during sequencing library preparations. Compared to ligase-mediated adapterization of nucleic acid samples, the direct incorporation of modified nucleotides conjugated to sequencing adapters increases the efficiency of library preparation and simplifies user work flows. Implementations also facilitate asymmetric adapterization of libraries, e.g., with different 5' and 3' adapters to permit production of stranded libraries and paired end sequencing.

Converting nucleic acid samples to sequencing-ready libraries includes a series of enzymatic manipulations to add oligonucleotide adapters that contain flow cell complementary sequences, primer binding sites, and indices. Depending on the particular sample preparation workflow, conventional adapterization may be inefficient. Therefore, new and more efficient sample preparation that limit sample loss would improve sequencing results, particularly for samples of limited quantity. Accordingly, the problem of complex and low yield sample preparations involving multiple DNA manipulations (end repair, A-tailing, and inefficient ligation) that each involve different inefficiencies is addressed by the direct incorporation of oligo-modified nucleotide analogues as provided herein. The modified nucleotides as disclosed herein able to undertake high efficiency intramolecular proximity ligation (either through reactive chemistry or otherwise) more efficiently than intermolecular classical ligation of adapters to sample DNAs. The proximity is mediated by the oligonucleotide adapters of the modified nucleotides, which can recruit or hold ligation reaction components in proximity to one another to improve the reaction efficiency. In some work flows as provided herein, certain biochemical steps in conventional workflows, such as ligation, can be eliminated completely. In one example, the problems of loss of sample due to incorrect combinations of adapters and loss of strandedness information are solved by sequential additions of 1) a first adapter via tagmentation followed by 2) a second adapter via polymerase-mediated adapter addition to yield fragments having asymmetric adapters.

The disclosed techniques provided increased yield during sample preparation relative to ligation-based steps. In certain embodiments, direct incorporation of oligo-modified nucleotide analogues using a polymerase decreases the number of workflow steps or sample manipulations to limit sample loss and increase user friendliness. Additional benefits include the ability to sequentially add asymmetric adapters (without accompanying sample loss), enable PCR or PCR-free sample preps, and retaining strandedness information. The disclosed method for 3' adapterization of nucleic acids provide advantages of more granular control of individual incorporation of the 3' adapter by a different technique relative to adding the 5' adapters in contrast to other methods where the 3' and 5' adapters are both added with the same method (i.e., dsDNA ligation).

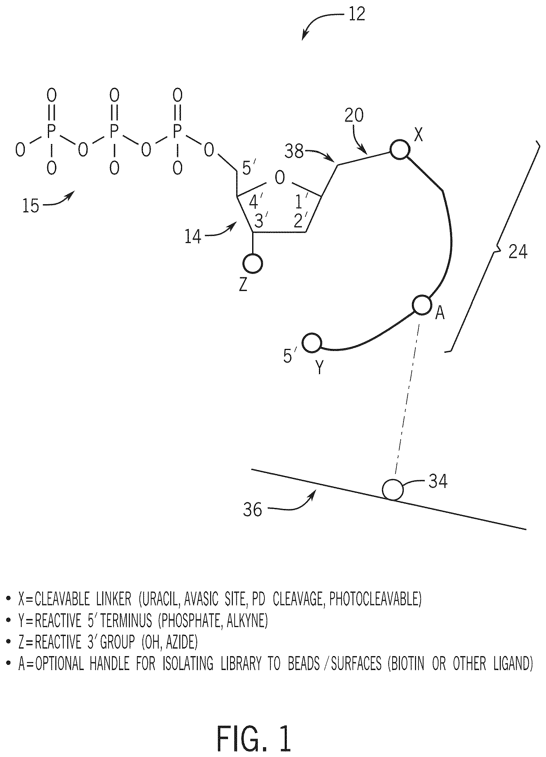

FIG. 1 is an example oligo-modified nucleotide analogue, also referred to herein as a modified nucleotide 12. In an embodiment the modified nucleotide 12 may be referred to as an oligo-modified nucleotide analogue (oNTP). The modified nucleotide 12 includes a pentose sugar, e.g., a deoxyribose 14 or a modified deoxyribose and a 5' phosphate group 15 (e.g., a triphosphate). Extending from a 1' carbon position of the deoxyribose 14 is a linker 20 that, in embodiments, may be a cleavable linker that is cleavable from the deoxyribose 14. The linker may include a nucleotide base group that will aid with basepairing during incorporation of the modified nucleotide. The base group can in turn have a cleavable group between it and the oligo group. The linker 20 may include a carbon or carbon chain comprising one or more intervening carbons, nitrogens, oxygens, or combinations thereof positioned before a cleave location, indicated by X. The linker 20 may include benzyl functional groups or a PEG spacer. The linker 20 may be chemically cleaved using tris(2-carboxyethyl)phosphine (TCEP) cleavage of a double bond or a tetrahydropyranyl (THP) cleavable moiety.

The linker 20 may be photocleavable or enzymatically or chemically cleavable. In an embodiment, a carbon-carbon bond of the linker 20 is cleaved by palladium-catalyzed cleavage. In an embodiment, the linker 20 includes a uracil at cleave location X, and the uracil is excised via a uracil DNA glycosylase (USER) to leave an abasic site at the cleave location X that can be cleaved by endonuclease VIII. In embodiments, the linker 20 includes one, two, three, four, five, six, or more carbons that separate the deoxyribose 14 from an oligonucleotide adapter 24. Shorter linkers 20 may leave a smaller "scar" upon incorporation of the modified nucleotide 12 into a nucleotide backbone as provided herein. Table 1 shows example cleavage chemistries that may be used in conjunction with the disclosed linkers 20.

**Table 1: Cleavage Chemistries**

| Cleave location x | Cleavage Chemistry | Cleavage Reagent | Compatibility |
|---|---|---|---|
| Disulfide | Chemical | Reductant | Use with compatible Z and/or Y chemistries |
| Allyl-T | Chemical | Pd complex | |
| Diol | Chemical | Periodate | |
| O-azidoethyl | Chemical | Reductant | Use with compatible Z and/or Y chemistries |
| 8-oxg G | Enzymatic | FPG | |
| Uracil | Enzymatic | USER enzyme mixture | |
| Restriction Site | Enzymatic | Restriction endonuclease | |

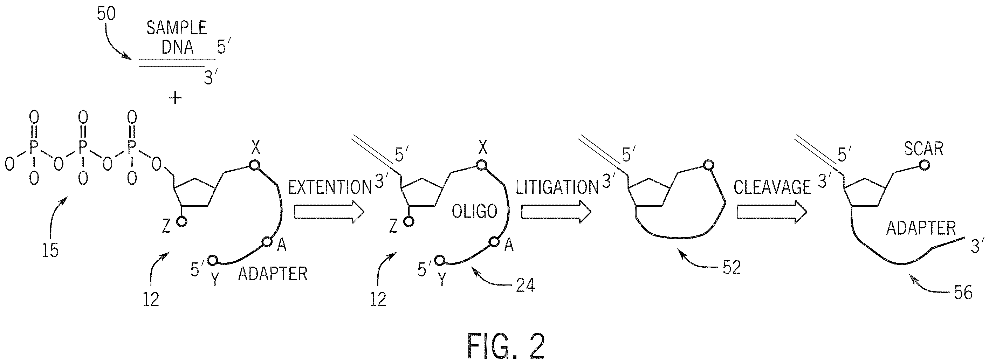

The oligonucleotide adapter 24 is coupled to and extends from the linker 20 and may include a single-stranded oligonucleotide or a partially double-stranded oligonucleotide, as disclosed herein. The oligonucleotide adapter 24 terminates at a 5' oligonucleotide end 30, indicated by Y. Cleaving at the cleave location X liberates a 3' end of the oligonucleotide adapter 24 (see FIG. 2). The oligonucleotide adapter 24 may be between 10-1000 nucleotides in length. In an embodiment, the oligonucleotide adapter 24 is between 10-100, 10-30, 30-50, or 30-100 nucleotides in length. Because adapter sizes vary depending on the library preparation and can be quite large, for example for PCR-free libraries, polymerase incorporation of modified nucleotides may decrease with large (e.g., greater than 1000 nucleotides) oligonucleotide adapters. However, incorporation of a 36-mer oligonucleotide adapter 24 of a modified nucleotide 12 was demonstrated to have high yield.

In an embodiment, the oligonucleotide adapter 24 may include one or more of the following: a barcode, an adapter sequence, a tag sequence, a primer binding sequence, a primer recognition sequence, a mosaic end sequence, a transposon recognition sequence, a capture site or capture sequence, a sequence complementary to a capture sequence, a unique molecular identifier (UMI) sequence, a restriction sequence, or an index sequence (e.g., a sample index sequence). The sequence of the oligonucleotide adapter 24 may be functional in a 5' to 3' direction in 3' adapterization embodiments as provided herein.

In an embodiment, the oligonucleotide adapter 24 may include or be coupled to an affinity binder, indicated as A, that functions as a handle to permit pulldown or isolation. For example, the affinity binder may be part of a binding pair, such as biotin/streptavidin or an antibody/antigen binding pair. The affinity binder A can be a first member of the binding pair, while a second member 34 of the binding pair can be coupled to a surface 36, e.g., a substrate surface, a bead surface, to permit isolation of the modified nucleotide 12, either before or after incorporation. In one embodiment, the modified nucleotide 12 is provided on the surface 36, and the disclosed adapterization or other incorporation steps occur on the surface 36. In one embodiment, the products of adapterization workflow steps are first generated and subsequently isolated on the surface 36. While the affinity binder is shown as being generally centrally positioned within the oligonucleotide adapter 24, it should be understood that the affinity binder may be coupled at other locations on the oligonucleotide adapter 24. Further, in embodiment, the oligonucleotide adapter 24 includes no affinity binder and/or the workflow steps as disclosed herein occur in solution or are uncoupled to a surface for to incorporate the modified nucleotide 12.

The modified nucleotide 12 includes a reactive 5' terminus, indicated as Y in FIG. 1. In embodiments, the reactive 5' terminus is a phosphate (e.g., a tri-phosphate) or an alkyne group reactive terminus. The modified nucleotide 12 also includes a reactive 3' group, indicated as Z, which may be a hydroxyl group or an azide. The reactive 3' group permits attachment of the 5' oligonucleotide end Y as generally disclosed herein, e.g., via ligation or a click reaction. One of more of X, Y, or Z reactive groups may be selectively blocked and deblocked. In one example, the Z reactive group may be reversibly blocked with a 3'-*O-*azidomethyl cap that is removable by tris(2-carboxyethyl)phosphine (TCEP) to regenerate a 3'-OH. The 5' reactive groups Y may be reversibly blocked by dephosphorylation and regenerated via phosphorylation steps. Table 2 shows example Z-Y ligation strategies. The disclosed strategies may include ligations with no deblocking, in which single extension occurs due to sterics. In other strategies, deblocking can be used to generate a reactive 3' group and/or a reactive 5' group.

**Table 2: Ligation strategies**

| Z | Y | Deblock | Ligation | Compatibility |
|---|---|---|---|---|
| 3'-OH | 5' phosphate | NA | Enzymatic | Can be single extension due to sterics of |
| | | | | modified nucleotide |
| 3'-azidoethyl | 5' phosphate | Reductant | Enzymatic | |
| 3'-o-NH₂ | 5' phosphate | Sodium nitrate | Enzymatic | |
| 3'-ester | 5' phosphate | Esterase(enzymatic) | Enzymatic | |
| 3'-azide | 5'-alkyne | NA | Cu-click | Cu-catalyzed ligation such that ligation does not occur before modified nucleotide incorporation |

The modified nucleotide 12 may be a modified purine or pyrimidine nucleotide. The modified oligonucleotide may be a uracil, thymine, cytosine, adenine, or guanine. In an embodiment, the nucleobase and the linker 20 may both be coupled to the 1' carbon position. In an embodiment, the nucleobase may be coupled to a carbon of the linker 20. In an embodiment, the linker 20 may extend directly from the nucleobase such that the nucleobase 38 is between the linker 20 and the deoxyribose 14. In an embodiment, the linker 20 of the modified nucleotide 12 may be a linker as set forth in (e.g., the linker 20 may have a chemical structure and may be arranged relative to and extend from the nucleobase 38 as in) U.S. Patent No. 9,127,314, which is incorporated by reference herein for all purposes.

Embodiments of the disclosure include compositions of modified nucleotides 12. The modified nucleotide 12 may be provided as a single modified nucleotide type (e.g., only one of uracil, thymine, cytosine, adenine, or guanine) or a mix of different nucleotides. For a particular reaction, all of the modified nucleotides 12 may have a same oligonucleotide adapter sequence or may have distinguishable oligonucleotide adapter sequences. Further, the modified nucleotide 12 may be provided in a reaction mixture together with unmodified nucleotides. It should be understood that certain features of the modified nucleotide 12 (e.g., the pentose sugar 14, the nucleobase 38) may be simplified for purposes of illustration in the disclosed embodiments.

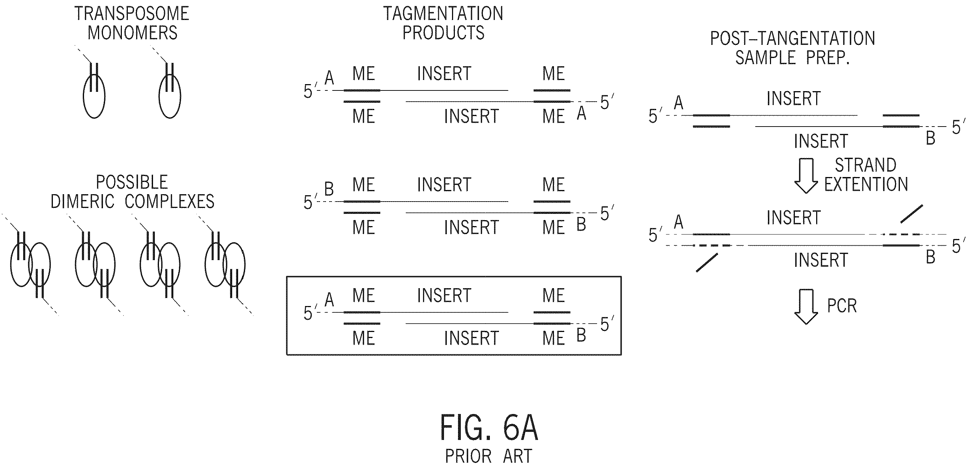

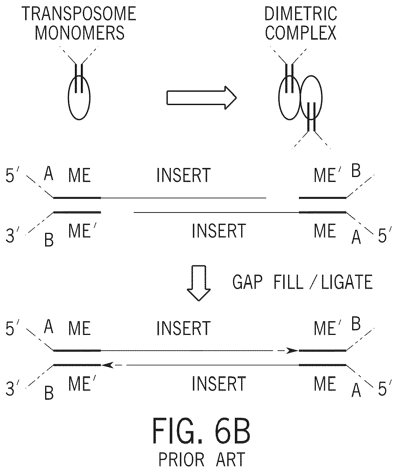

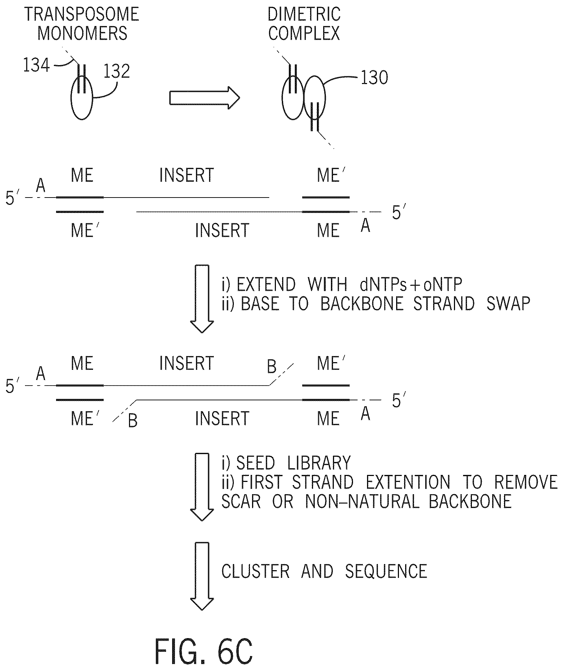

FIG. 2 is an example process for incorporating the modified nucleotide 12 onto a 3' end of a sample DNA molecule 50, shown as a double-stranded DNA. The sample DNA molecule 50 may be a DNA fragment generated by suitable fragmenting techniques (enzymatic fragmenting, sonication, natural fragmentation of cell free DNA). In an embodiment, the fragments may be generated by transposome-mediated 5' adapterization (see FIG. 6). Thus, the sample DNA may include 5' adapters in some embodiments.

At a first step, the sample DNA 50 is contacted with the modified nucleotide 12 in the presence of a polymerase to extend the 3' end via incorporation of the modified nucleotide 12 at the 5' phosphate group 15. The polymerase may be a permissive polymerase (e.g., sequencing-by-synthesis polymerase capable of 3' addition of dye-conjugated nucleotides). Further, the polymerase may lack 3' to 5' exonuclease activity to prevent proofreading excision of the modified nucleotide 12. For extension, as shown in FIG. 2, the 5' triphosphate group 15 of the modified nucleotide 12 is reacted with the 3' end of the sample DNA 50. The single-stranded oligonucleotide adapter 24 of the incorporated modified nucleotide 12 extends from the 3' end of the sample DNA 50. The 5' oligonucleotide end Y is ligated to the reactive group Z, e.g., via enzymatic or chemical ligation, which causes the oligonucleotide adapter 24 to form a loop 52. In an embodiment, enzymatic ligation is mediated by DNA ligase, such as a template-independent T4 ligase reaction, or a specialist single stranded ligase such as Circ ligase. In an embodiment, chemical ligation is mediated by copper-catalyzed azide-alkyne cycloaddition (CuAAC) of a click reaction.

Cleavage at the cleavage site X liberates a 3' end of the oligonucleotide adapter 24, while leaving a "scar". While incorporation of the modified nucleotide 12 at a single 3' end is shown, it should be understood that the illustrated reaction may occur in parallel at both 3' ends of the double-stranded sample DNA 50. It was demonstrated that polymerases were able to read through a non-natural backbone connection created using the azide-alkyne click reaction. Thus, the scar and, in some cases, any non-natural 3' linkage (i.e., click chemistry) can be retained without significantly impacting subsequent polymerase/amplification reactions.

The polymerase-mediated incorporation of the modified nucleotide 12 results in formation of a 3' adapterized DNA 56 via the addition of nucleotides in the oligonucleotide adapter 24 at the 3' end of the sample DNA 50. Thus, a relatively simple and efficient reaction permits the batch addition of up to hundreds of nucleotides to a 3' end of a nucleic acid via a single polymerase incorporation. As discussed herein, selective 3' addition of nucleotides separated from a 5' adapterization facilitates a sequencing workflow using asymmetric adapters, such as a paired end sequencing workflow that sequences forward and reverse strands or tagmentation where the 5' end is added by the transposase. Further, while relatively longer oligonucleotides may be ligated onto a strand end, such ligations are relatively inefficient as compared to polymerase reactions. Thus, using the disclosed techniques, more efficient addition of adapters to 3' ends is achieved.

In the illustrated embodiment, the sample DNA 50 includes a 5' overhang. However, the DNA 50 may be provided as blunt or with 3' overhangs. The polymerase extends the 3' end to pair with the 5' overhang and using a nucleotide that is complementary to a nucleotide of the 5' overhang. The 5' overhang may be a single base overhang. However, as discussed herein, the 5' overhang may represent an already-incorporated 5' adapter. In an embodiment, the modified nucleotide 12, prior to cleavage and liberation of the 3' end, is a reversible extension terminator. That is, the structure of the modified nucleotide 12 acts to prevent subsequent addition of modified nucleotides 12 or unmodified nucleotides via reaction of a 5' terminus with the reactive group Z. In an embodiment, to prevent undesired extensions, the reactive group Z may be reversibly blocked as provided herein.

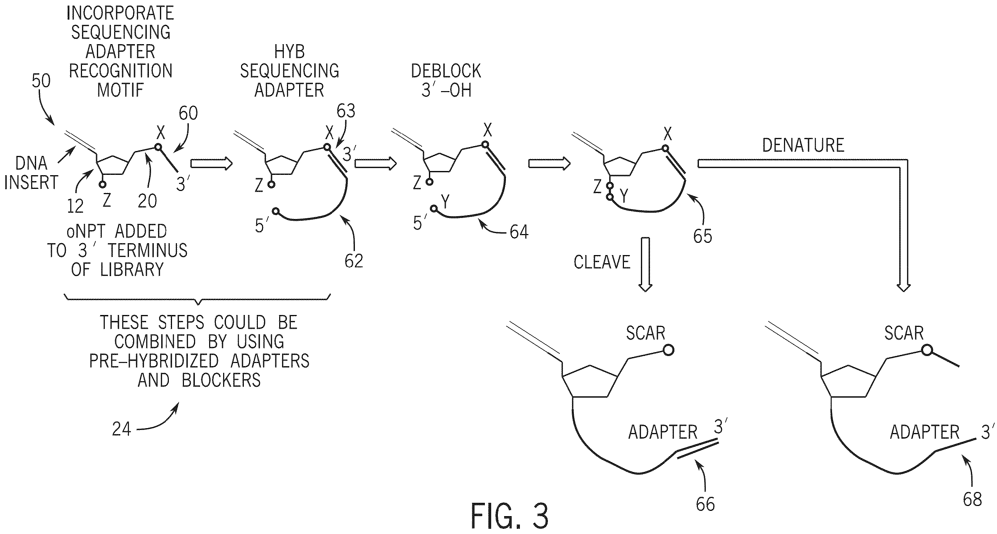

FIG. 3 is a schematic illustration of a modified nucleotide 12 that includes a hybridized oligonucleotide adapter 24. The oligonucleotide adapter 24 includes a first strand 60 that is directly coupled to (e.g., covalently bound to) the linker 20. The first strand 60 hybridizes to a partially complementary second strand 62 such that the oligonucleotide adapter 24 includes a partially double-stranded portion 63 as well as a single-stranded portion 64. The single-stranded portion 64 includes a 5' reactive terminus Y.

The formation of the oligonucleotide adapter 24 as an assembly may occur after incorporation of the modified nucleotide 12 that includes only the first strand 60. That is, the polymerase-mediated incorporation may be followed by an annealing step at temperatures that permit specific hybridization and that are selected to be sufficiently high to prevent nonspecific binding. In other embodiments, the modified nucleotide 12 may be provided with the oligonucleotide adapter 24 pre-formed and including the first strand 60 and the second strand 62. The 3' terminus of oligonucleotide 63 can be blocked such that extension does not occur during incorporation of modified nucleotide 12. The reactive 3'-Z can be reversibly blocked such that incorporation terminates the sample DNA 50 and/or to prevent undesired side reactions of the single-stranded portion 64 before incorporation.

Ligation of the 5' oligonucleotide end Y with an active (or de-blocked) 3' reactive group Z forms a loop 65 that is mostly single-stranded, but that include the 3' double-stranded portion. The 3' end of the second strand 62 can be liberated by one or both of a cleavage step or a denaturing step. Cleavage yields a modified nucleotide 12 with a double-stranded end 66, which can be removed in subsequent denaturing steps (e.g., for amplification). Denaturation without cleavage yields a modified nucleotide 12 with a single-stranded end 68 and a single-stranded scar.

Accordingly, certain embodiments of the disclosure include incorporation of the modified nucleotide 12 with the oligonucleotide adapter 24 linked to a 1' position via a linker 20 and having a terminal 5' end. The oligonucleotide adapter 24 can be coupled to an available reactive Z group via the 5' end and cleaved to release the 3' end. Thus, the disclosed techniques include transitioning the oligonucleotide adapter free between a terminal 5' end when coupled to the 1' position of the deoxyribose 14 and a terminal 3' end when coupled to the 3' position of the deoxyribose 14. However, in certain embodiments, the oligonucleotide adapter 24 may be linked at the 1' position and have a terminal 3' end that does not react with the Z group and/or undergo cleavage.

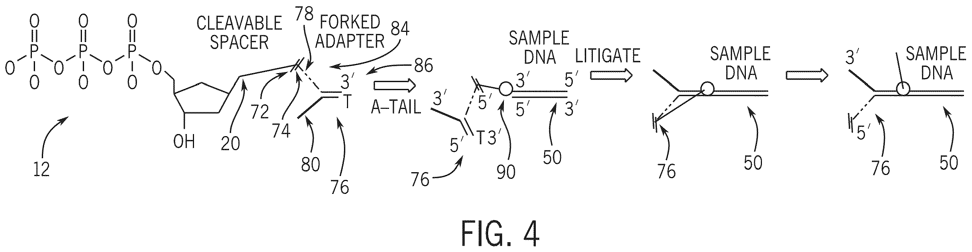

FIG. 4 illustrates an approach in which conventional adapterization via ligation is enhanced using the modified nucleotide 12 that includes an oligonucleotide adapter 24 that forms a forked adapter. The illustrated modified nucleotide 12 is coupled at a cleavage site 72 to a forked adapter 76. The cleavage site 72 is coupled to a terminus 74 of a first fork 78 of the forked adapter. The first fork 78 includes a first adapter that is non-complementary to a second adapter on the second fork 80. Thus, in an example in which the sample DNA 50 is adapterized to include different end adapters (e.g., A and B adapters), the forked adapter 76 carries both of the different adapters on different forks such that ligation of the forked adapter 76 to the sample DNA 50 adds both adapters at once and creates a forked end. In an embodiment, the forked adapter 76 may be a Nextera adapter (Illumina, Inc.) that includes a P5 and i5 sequence on the first fork 78 and a P7 and i7 sequence on the second fork 80. The forked adapter 76 may be a universal adapter, such that different sample DNA fragments all are adapterized with forked adapters 76 having a common sequence.

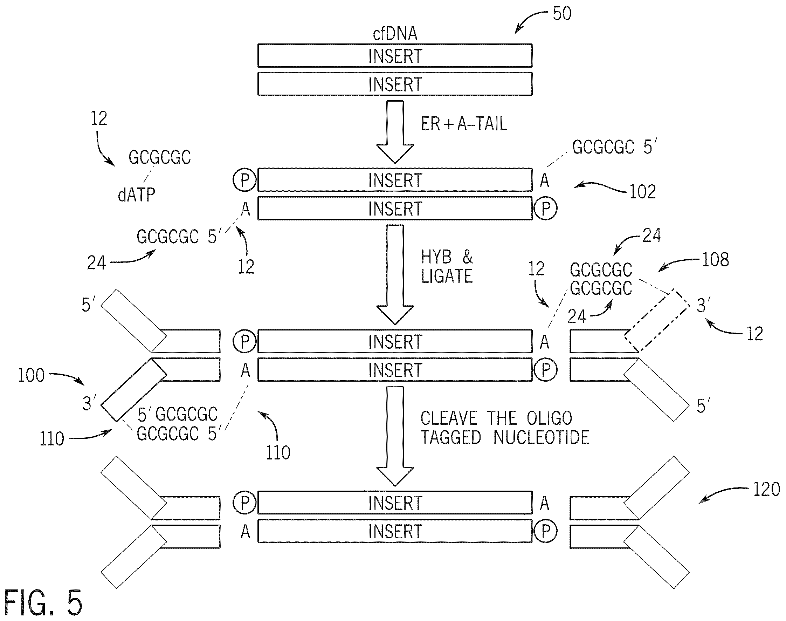

The forked adapter 76 is covalently, and cleavably, linked to the linker 20 of the modified nucleotide 12. However, in embodiments, the forked adapter 76 may hybridize to a complementary oligo extending from the cleavable linker 20 (see FIGS. 2 and 5). The forked adapter includes a double-stranded portion 84 including a 3' T overhang 86. The T-overhang 86 will eventually be ligated, e.g., via T4 ligase, to an A-tailed sample DNA 50. The A-tail consists of the modified nucleotide 12, which is a modified adenosine. Thus, the incorporation of the adenosine modified nucleotide 12 onto the 3' end of the double-stranded sample DNA 50, modified by the forked adapter 76 having a T overhang 86, creates the A-tail that is used in the subsequent ligation of the T-overhang 86.

In the depicted embodiment, the double-stranded sample DNA 50 is blunt-ended, and incorporation of the adenosine modified nucleotide 12 into the double-stranded sample DNA 50 creates a 3' A tail 90 from which the forked adapter 76 extends. The A-tailing may be performed by a compatible A-tailing polymerase, such as taq polymerase, klenow, or a terminal transferase. The A-tail 90 creates a substrate for ligation of the 3' T 86 of the double-stranded portion 84. The forked adapter 76 extends from the cleavable linker 20 of an incorporated adenosine, and the ligation efficiency of these elements is enhanced by the coupling, e.g., by holding of the elements in proximity to one another in advance of and during ligation. Thus, the ligation occurs while the forked adapter 76 is still coupled to the cleavable linker 20.

After ligation, the hold of the forked adapter 76 via the cleavable linker 20 to the incorporated adenosine can be reversed, leaving a scar at an internal site that corresponds to the location of the incorporated modified nucleotide 12. The sample DNA 50 includes the forked adapter 76, added via ligation. While only one end of the sample DNA 50 is illustrated, it should be understood that the illustrated adapterization may occur at both ends of a fragment of sample DNA 50 and as part of preparation of a sequencing library.

FIG. 5 shows an approach that uses a modified nucleotide 12 (here, modified adenosine) that includes an oligonucleotide adapter 24 that recruits the forked adapter 100 via hybridization. Here, the sample DNA 50 is shown as a cell free DNA fragment. However, other sample DNA formats are also contemplated. End repair and A-tailing using the modified nucleotide 12 adds a terminal adenosine 102 including the oligonucleotide adapter 24 at the 3' ends of the sample DNA 50. The forked adapter 100 may include a T overhang to facilitate ligation of a double-stranded portion of the forked adapter 100 to the A-tailed sample DNA 50. For the approaches herein, e.g., as in FIG. 4 and FIG. 5, the ligation step can be either enzymatic or chemical.

The forked adapter 100 also includes a modified nucleotide 12 at respective 3' noncomplementary ends of the forked adapter 100. Adding the forked adapter 100 to reaction mixture including the sample DNA 50 brings respective oligonucleotide adapters 24 extending from both the 3' A Tail 102 and from the forked adapter 100 in proximity to one another. The oligonucleotide adapters 24, as illustrated, are self-complementary and can hybridize to form a double-stranded hybridized adapter assembly 108. This assembly 108 adds stability to and promotes the ligation of the forked adapter 100 to increase ligation reaction efficiency by holding the forked adapter 100 in proximity to the A-tail 102. The hybridization to form the double-stranded hybridized adapter assembly 108 may occur prior to and/or during ligation. In an embodiment, the oligonucleotide adapter 24 of the A-Tail 102 and of the oligonucleotide adapter 24 of the forked adapter 100 can be a same sequence that has high self-complementarity. In the depicted embodiment, the six 5'-most nucleotides are self-complementary such that the 5' portion of a first oligonucleotide adapter 24 is a reverse complement of at least the first six nucleotides of a second nucleotide adapter 24. The self-complementary region may include at least 4, 5, 6, 7, 8, 9, 10, 11, 12, 13, 14, 15, or more nucleotides, and may be positioned at a 5' end, or may be an internal region. Further, the nucleotide adapter 24 may include one or more self-complementary regions.

To complete preparation of the adapterized sample DNA for subsequent target enrichment and/or amplification steps, the oligonucleotide adapters 24 can be cleaved at their respective cleave sites 110 of the incorporated modified nucleotides. Cleavage yields an adapterized double-stranded fragment 120 including ligated forked adapters 100 at both ends. The fragment 120 may be part of a sequencing library using in a sequencing reaction. The fragment 120 may be provided to subsequent steps, e.g., amplification, enrichment, or direct to sequencing or amplification-free sequencing steps.

Each 3' end of the double-stranded fragment 120 includes respective scars from remaining portions of the linker at each modified nucleotide site. Thus, for an individual strand of the double-stranded fragment 120, a first scar is present at the internal site corresponding to the A-tail 102 and a second scar is present at the 3' end of the forked adapter 100. The two scars are present on each strand. However, in embodiments, the scars are eliminated at subsequent amplification or extension steps. Further, only the scar at the A-tail 102 is covered by a polymerase for subsequent processing. The scar on the forked adapter 100 can be beyond the adapter sequence and may not be copied across/covered by a polymerase.

FIGS. 6A-B are schematic illustration of prior art tagmentation workflows. Using transposome-based strategies for 5'-adapterization. FIG. 6C shows a tagmentation workflow using modified nucleotides as disclosed herein. Compared to current transposome-based sample preparations, 3-adapterization with modified nucleotides 12 offers several advantages.

An example Nextera^{™} or Illumina DNA prep (Illumina, Inc.) sample preparation (FIG. 6A) can employ two different transposons. Because transposomes are dimeric complexes, only 50% of dually transposed library fragments have both adapter types and are sequencing compatible. In tagmentation with forked adapters (FIG. 6B), each tagmentation event introduces a 5' A-type adapter, and 3' adapterization is then accomplished via an extension ligation step. However, in practice this extension ligation step has low yields because only a portion of the products include the desired dual adapter types.

Efficient incorporation of modified nucleotides 12 including oligonucleotide adapters 24 has been demonstrated. In an embodiment, the modified nucleotides 12 as provided herein are used in polymerase-mediated adapterization to improve the yield of sequence-able fragments in sequencing library preparation, as shown in FIG. 6C. Sample preparations using the modified nucleotides 12 can employ a single transposome type for 5' adapterization followed by 3'adapterization with modified nucleotides 12 in principle generates only A-B adapterized libraries. Thus, the simplified transposon design in FIG. 6C does not require forked adapters or strategies to selectively denature ME' and re-anneal a new fragment.

In the example shown in FIG. 6C, the transposome complex is a homodimer 130 in which the monomers 132 include only a single adapter type, a 5' adapter or A adapter 134 in the illustrated embodiment. Tagmentation using the homodimer 130 adds the 5' adapters, and the double-stranded ME (mosaic end) sequence to 5' ends of the insert. At a next step, a modified nucleotide 12 carrying a 3' or B adapter can be incorporated into the 3' ends. This may be done with a mix of 4 different modified nucleotides 12 for each base in the template strand, or with various mixes of natural nucleotides and modified nucleotides 12.

Ligation to a 3' reactive group and linker cleavage results in the base-to-backbone swap of the B adapter so that the 3' ends of the inserts are extended by the B adapter sequence. The B adapter is non-complementary to the 5' adapter in embodiments. Subsequent steps after the asymmetric adapterization can include first strand extension to remove the scar or non-natural backbone, and clustering and sequencing of the forward and reverse strands.

FIGS. 1-5 and 6C show different arrangements of oligonucleotide adapters 24 that are used to form and/or recruit end-adapters in sequencing library preparation. In certain embodiments, the sequential addition of adapters enables creation of asymmetric libraries or dumbbell libraries. For example, the 3' adapterization via modified nucleotides 12 can be used subsequent to a 5' adapter addition. A dumbbell library consist of double-stranded nucleic acid fragments with hairpin adapters at both ends. The dumbbell structure permits sequencing around the entire dumbbell via a strand-displacing polymerase with a resulting sequence that represent both the sense and antisense strands. The dumbbell-based amplification may also be a rolling circle amplification, which generates multiple copies of the insert. In one example, a dumbbell sequencing hairpin adapter may be coupled (via the linker) to a modified nucleotide 12 that is added by A-tailing (see FIG. 4) to a double-stranded nucleic acid fragment. The oligonucleotide adapter 24 may form a hairpin, whereby a 5' end of the hairpin, after incorporation of the associated nucleotide, is ligated to a 3' reactive group. Cleavage from the linker liberates the 3' end of the hairpin. The 3' end of the hairpin can in turn be ligated to a free 5' end of the fragment. Ligation of the coupled hairpin adapter is improved because the hairpin adapter extends from an incorporated 3' modified adenosine that is added by polymerase.

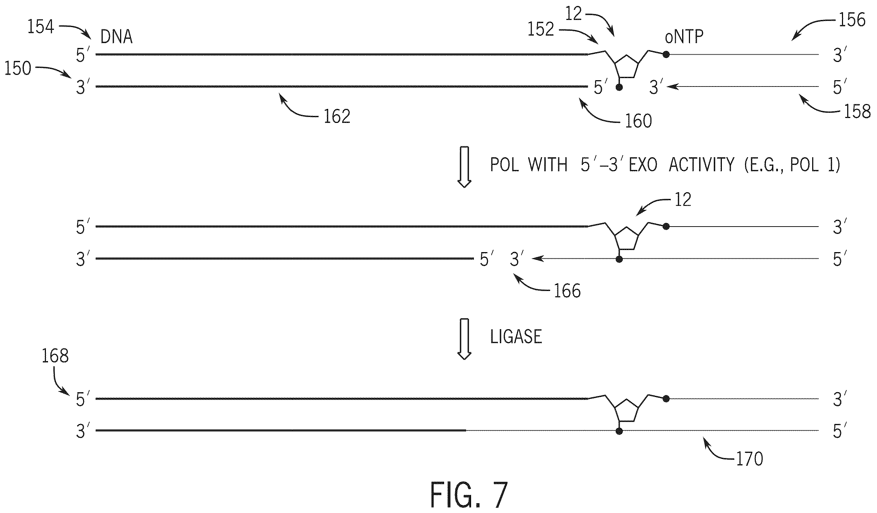

FIG. 7 shows an adapter primer extension across the modified nucleotide 12 using a polymerase with 5' to 3' exonuclease activity. In the illustrated example, the double-stranded nucleic acid 150 has the modified nucleotide 12 already incorporated via addition at a 3' end 152 of a first strand 154 such that the first strand includes the oligonucleotide adapter 156, shown here as a single-stranded oligonucleotide. Incorporation of the modified nucleotide 12 may occur as generally discussed herein. In an embodiment, the oligonucleotide adapter includes a free 3' end that may be generated via cleavage as discussed herein, or the oligonucleotide adapter may be coupled to the modified nucleotide 12 such that the 3' end is free, as in the illustrated embodiment. The adapter 156 may be single-stranded, and to generate a double-stranded adapterized fragment, an adapter primer 158, complementary to the adapter 156, is extended using a polymerase. Instead of using a strand displacing polymerase to extend from the adapter primer 158 across the modified nucleotide 12, a polymerase with a 5'-3' exonuclease activity extends the adapter primer 158 towards a 5' end 160 of a second strand 162 of the nucleic acid 150 and across and beyond the modified nucleotide 12. By continuing with the extension, a nick 166 in the nucleic acid 150 is shifted via 'nick translation' that is followed by a ligation to seal the nick to generate a nucleic acid 168 that incorporates a double-stranded adapter 170 (e.g., the adapter 156 and its complement adapter primer 158) on at least one end. The advantage of the illustrated embodiment is the nick 166 is separated from or moved away from a location of the modified nucleotide 12 so that structural differences of the modified nucleotide 12 relative to an unmodified nucleotide do not inhibit ligation at the nick 166. Accordingly, one or more adapters, e.g., sequencing adapters, may be incorporated onto a nucleic acid fragment end or ends via the illustrated workflow that includes modified nucleotide addition, primer extension with exonuclease activity, and subsequent ligation.

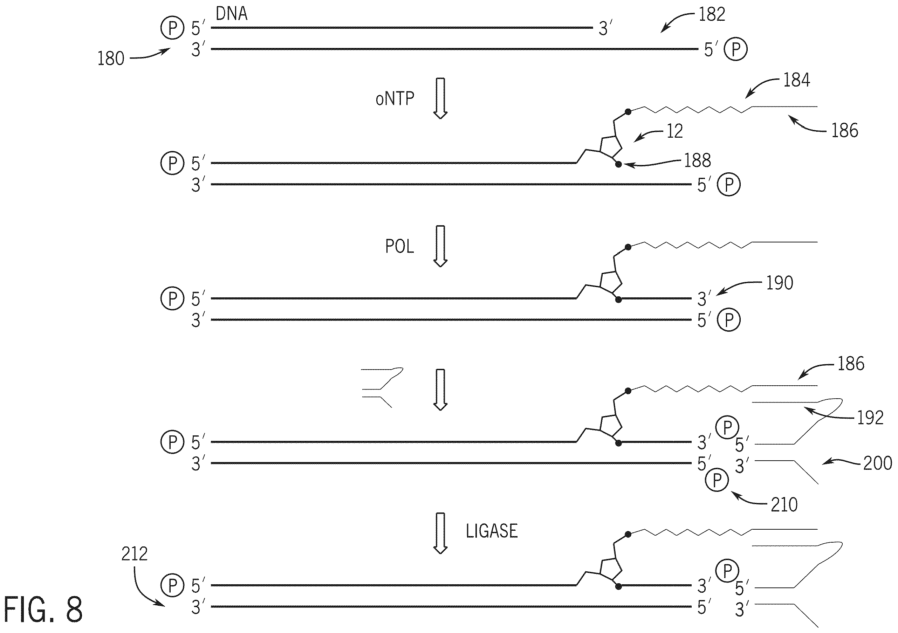

FIG. 8 shows an embodiment in which the modified nucleotide 12 is incorporated at a 3' recessed end including of a double-stranded DNA fragment 180. The modified nucleotide is added to pair with an oligonucleotide in the 5' overhang 182. Addition of the modified nucleotide 12 also incorporates the linked oligonucleotide adapter 184 including a single-stranded lure site 186. Here, the oligonucleotide adapter 184 is linked to the 1' position, and the 3' position 188 is available for initiation of dNTP addition. A polymerase then adds dNTPs and extends to flush out the 3' recess, using the overhang 182 as the template. The polymerase-extended 3' end 190 may be blunt or include an overhang. The oligo portion of the modified nucleotide 12, which includes the lure site 186, next functions as a 'lure' to which an adapter 200 is hybridized via a complementary sequence 192. This anchors the adapter 200 close to the template 180, which promotes a more efficient ligation reaction at the illustrated blunt end 210 to append the adapter 200 to the template to generate an adapterized fragment 212.

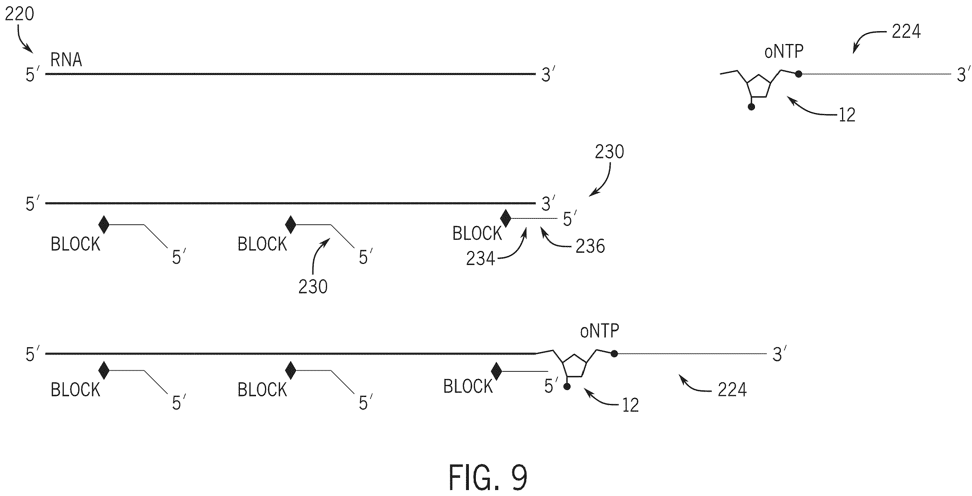

FIG. 9 shows an embodiment in which the modified nucleotide 12 is incorporate at the 3' end of an RNA molecule 220. Ordinarily, modified nucleotides do not get incorporated to the ends of single stranded nucleic acids. However, by contacting the single-stranded RNA 220 with single-stranded oligos 230 having a mixture of 3' end portions 234 that are random in base composition (e.g., a hexamer end portion 234) or with a poly-T portion and that are blocked from extension (e.g., via a 3' end block on the oligo 230), and a 5' end portion 236 with a known sequence, a 'splint' can be hybridised to the RNA 220. The set of oligos 230 having a mix of different random sequence random portions 234 can hybridise at any position along the molecule and based on the sequence of the particular random end portion 234. However, when a particular oligo 230 hybridises at the 3' end of the RNA molecule 220 based on the random portion 234 having complementary with the 3' end portion of the molecule 220, an overhang of the 5' end portion 236 with a known sequence is created. This overhang permits incorporation of the modified nucleotide 12 at the 3' end. Examples of the sequence of the splint include: 3' block-nnnnnn - TTTTT-5', where 'n' is any base, including inosine. In this particular example the modified nucleotide 12 would be derived from adenosine base to promote complementary binding to the splint oligo 230.

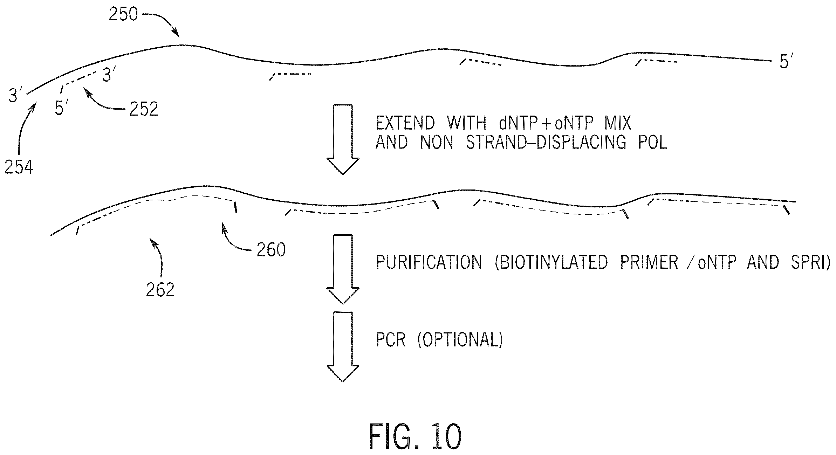

FIG. 10 shows an example in which the modified nucleotides 12 are used in extension reactions. A template strand 250 is primed with primers 252 that include randomers having 5' adapters 254. However, in embodiments, the primers 252 may be targeted primers, e.g., for targeted sequencing reactions. Extending using a reaction mix of unmodified nucleotides and modified nucleotides results in termination of extension upon incorporation of modified nucleotides 12. Each modified nucleotide can be coupled to a 3' adapter 260 as provided herein. Thus, the extension products 262 have asymmetric adapters 254, 260 at their ends. This technique eliminates the need for end repair and ligase-catalyzed adapterization. In addition to eliminating the need for multiple enzymatic adapter ligation steps, this approach also has the advantage eliminating the need for fragmentation and the associated post-fragmentation size selection. That is, the combination of randomer primer annealing and termination based on modified nucleotide incorporation forms separations between the extension products 262. While priming and extension from a single strand is shown, it should be understood that the reaction may occur on both a forward and reverse strand.

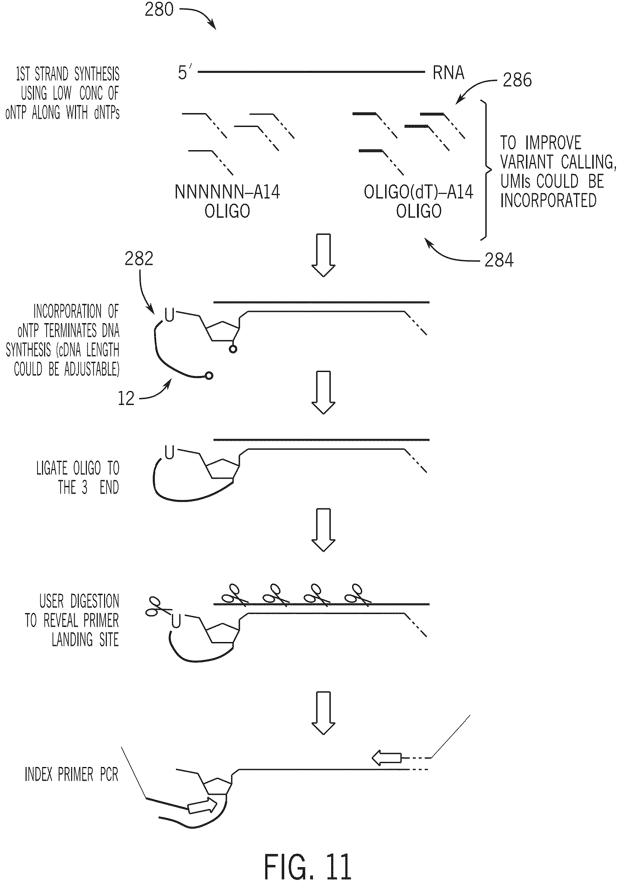

FIG. 11 shows an example in which the modified nucleotides 12 are used for RNA sequencing library preparation, e.g., from a single-stranded RNA molecule 180. Use of modified nucleotides 12, carrying a 3' adapter 282, permits 3' adapter addition directly in a first strand cDNA synthesis step. The workflow would be conducted as follows. A reverse transcriptase would extend off of primers 284 containing a 5' adapter 186, shows as an A14 oligo, linked to random hexamers or oligo(dT) (for binding of poly A RNA tails). The reaction mixture includes modified nucleotides 12 supplemented at low concentration along with dNTPs. The modified nucleotides 12, once incorporated, would terminate extension. Thus, the modified nucleotide 12 concentration could be adjusted to alter the length of the cDNA (e.g., higher concentrations of modified nucleotides 12 would yield shorter cDNA products). The modified nucleotide 12 incorporated-cDNA could be then captured using a biotin handle for purification and buffer exchanges. The 3' adapter 282, potentially B15, would then be linked to the 3' end by proximity-enhanced ligation and revealed through a USER cleavage step that liberates a 3' end. Once the 3' oligo is cleaved, an index primer PCR would amplify the cDNA. The sequential addition of adapters to the same strand means this library preparation inherently enables determination of sense/antisense strand information in the final sequencing library. This approach also eliminates the need for complicated template switching protocols.

Variations of this approach could also be used for targeted RNA-seq applications such as splice variant and gene fusion analysis and whole exome sequencing. Compared to other approaches where two probes and a ligation event are required (eg RASL-seq for gene fusions), enrichment, which requires an additional step, or multiplex PCR, which requires an additional step and two primers for each target, this approach combines sequence specific binding with library adapterization. Because adapters are added sequentially, steps downstream from the initial stranded extension with modified nucleotides 12 can be performed on a bead (e.g., streptavidin bead). This could increase efficiency for downstream processes due to the high concentrations of adapters that could be co-immobilized in the same space as the singly-adapterized library.

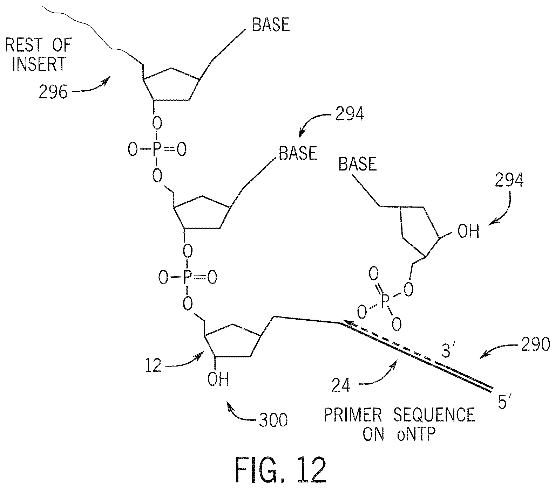

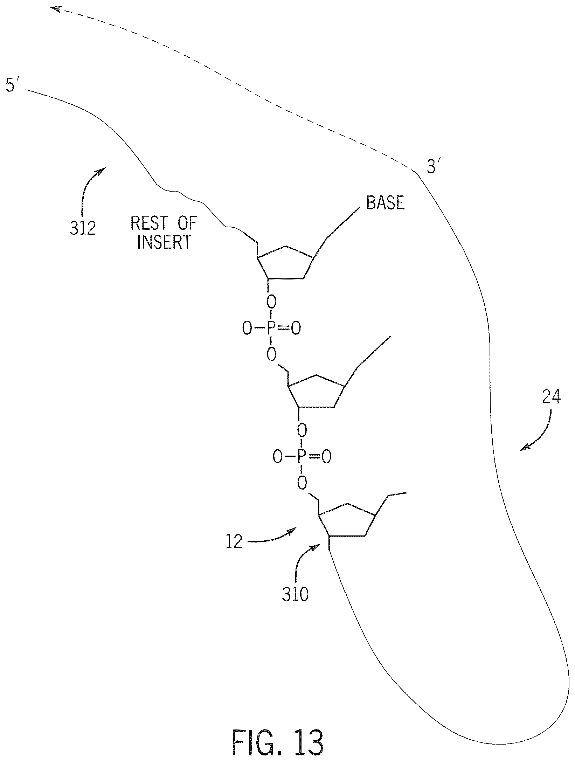

Depending on the polymerase employed, it is possible that the oligonucleotide adapter 24 of the modified nucleotide 12 may not need to be ligated to the 3'-OH to function as an adapter, as shown in the example of FIG. 12. In the illustrated example, the oligonucleotide adapter 24 is partially double-stranded and includes or is hybridized to a primer 290 that can be extended in a 3' direction. In the illustrated embodiment, the addition of bases 294 based on the template 296 "jumps" the modified nucleotide 12. However, depending on the arrangement of the modified nucleotide 12, the extension may use the base associated with the modified nucleotide 12 as a template. As illustrated, the 3'-OH reactive group 300 is not ligated directly to an end of the oligonucleotide adapter 24, and there is no base-to-backbone swap in the illustrated example. FIG. 13 shows an arrangement in which the base-to-backbone swap at the reactive 3'-OH 310 has occurred, and the oligonucleotide adapter 24 forms a loop (or hairpin) that can be extended in the 3' direction to copy a single-stranded template molecule 312.

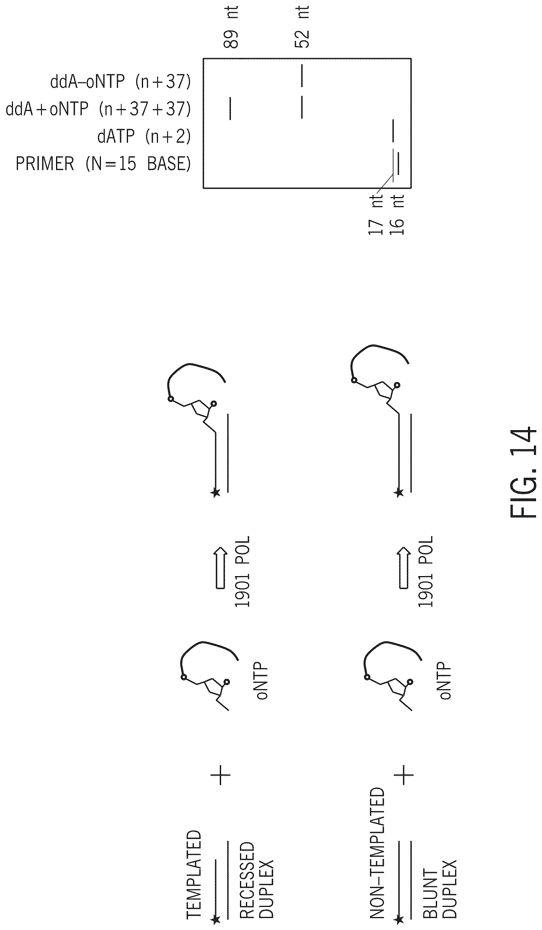

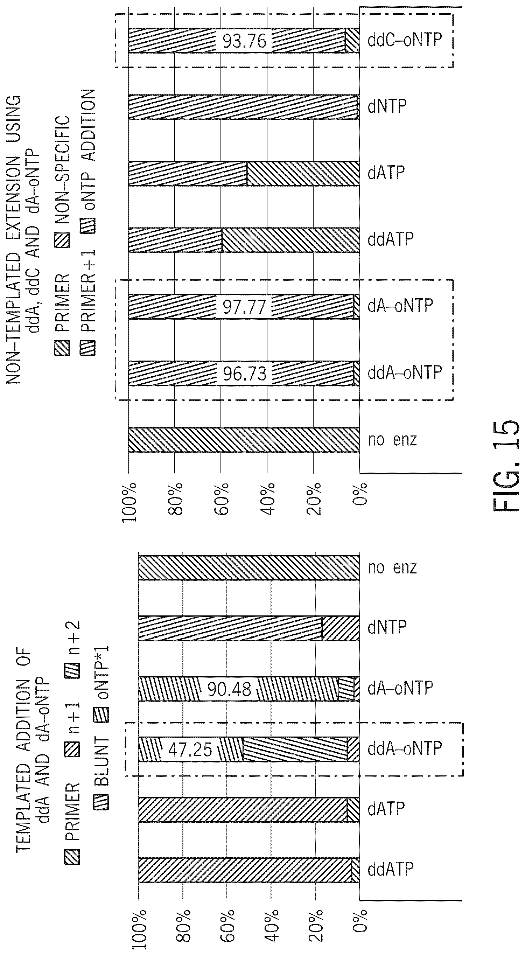

FIG. 14 is an illustration of a reaction conducted to incorporate modified nucleotide incorporation onto blunt and recessed duplex DNA (left) and the expected resultant product sizes (right). FIG. 15 shows results of modified nucleotide incorporation onto blunt and recessed duplex DNA according to the reaction illustrated in FIG. 14. Modified nucleotides, oNTPs, were incorporated by 1901 pol in both templated and non-templated reactions with about 90% template incorporation and about 95% non-templated incorporation by 5 minutes.

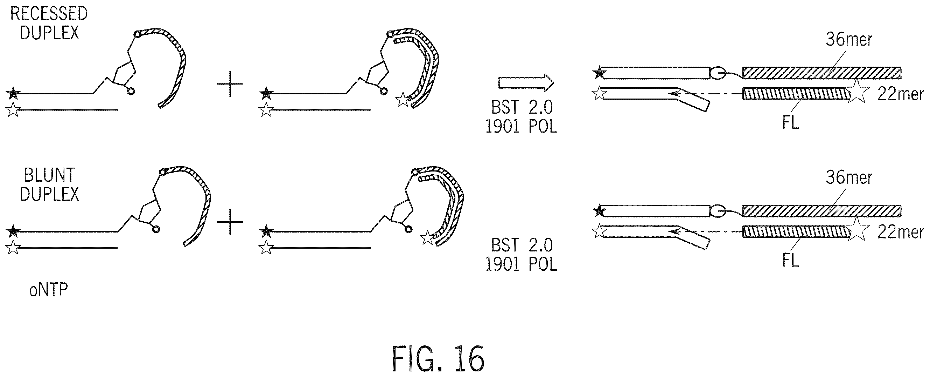

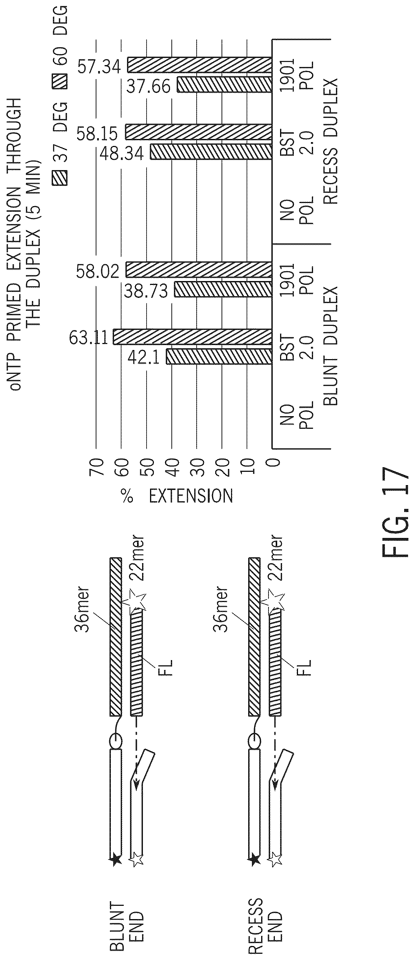

FIG. 16 is an illustration of modified nucleotide primed extension onto blunt and recessed duplex DNA, and FIG. 17 shows results of modified nucleotide primed extension onto blunt and recessed duplex DNA. In the reaction, a single-stranded oligonucleotide adapter was incorporated onto a blunt or recessed duplex fragment at a 3' end. The single-stranded oligonucleotide binds via sequence complementarity to a primer. The primer was extended using 1901 pol or Bst 2.0 and strand displacement of the duplex during primer extension occurred. That is, the displaced strand was the strand without the incorporated modified nucleotide. Bst 2.0 pol exerted highest activity in both blunt and recess duplex-strand displaced extension by 5mins at 60° incubation.

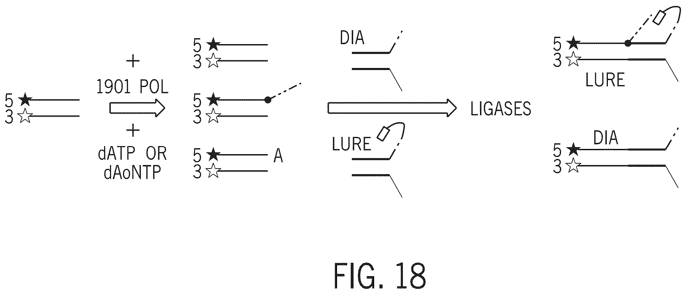

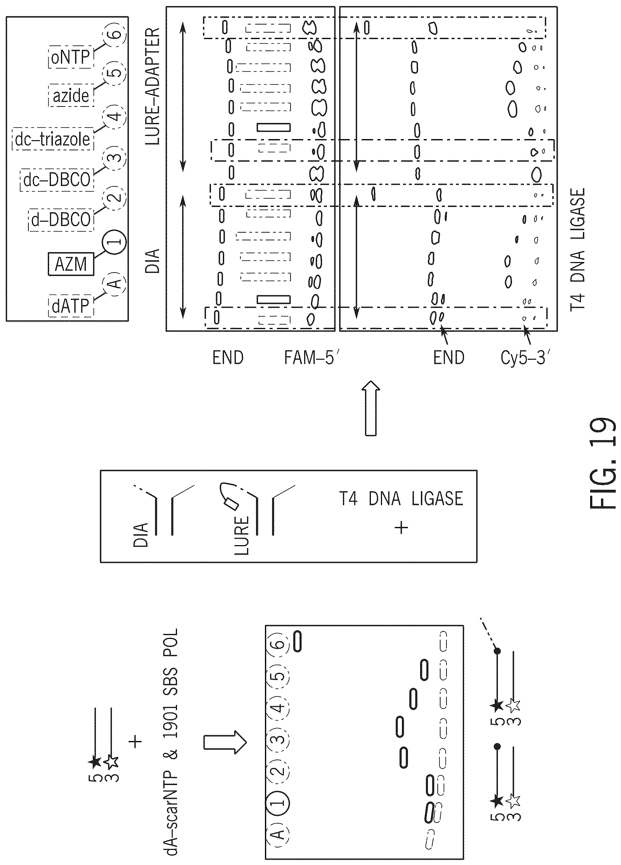

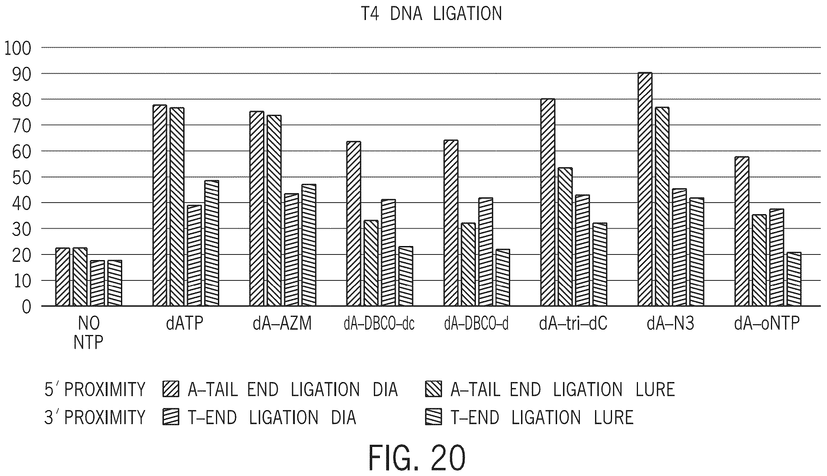

FIG. 18 is an illustration of adapter ligation onto blunt duplex DNA including a modified nucleotide. Either a modified adenosine including a lure or a dATP control was incorporated at the end of a duplex. FIGS. 19-20 shows results of a lure-based adapter ligation relative to ligation with other nucleotide ends. While ligation including the lure tail was at a lower preference relative to other ends, ligation was demonstrated to occur. T4 ligase showed the best ligation in the presence of the oNTP lure tail.

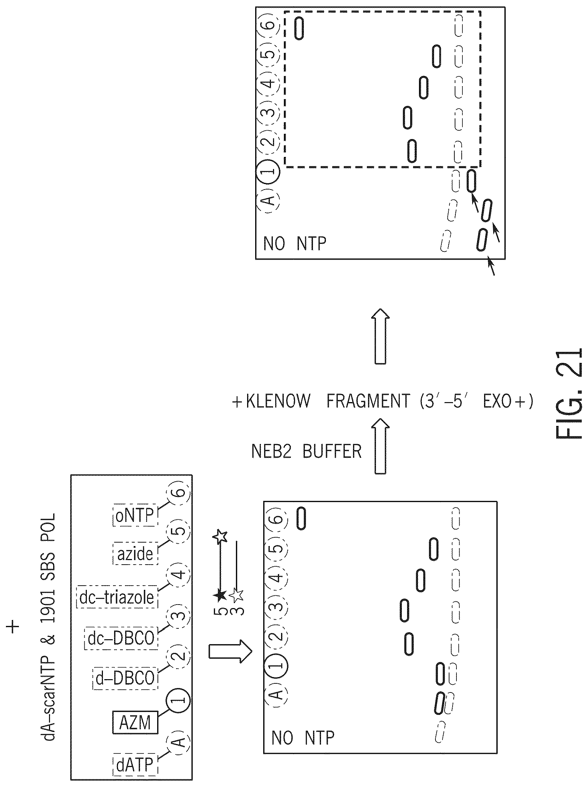

FIG. 21 shows exonuclease inhibition based on modified nucleotide scar size. 3' - 5' exonuclease activity can vary largely from the dNTP type and base modification. The size of the scar can be inversely correlated to ligation efficiency.

The disclosed techniques may be used to modify a nucleic acid sample or a sample nucleic acid. The sample nucleic acid can be derived from any in vivo or in vitro source, including from one or multiple cells, tissues, organs, or organisms, whether living or dead, or from any biological or environmental source (e.g., water, air, soil). For example, in some embodiments, the sample nucleic acid comprises or consists of eukaryotic and/or prokaryotic dsDNA that originates or that is derived from humans, animals, plants, fungi, (e.g., molds or yeasts), bacteria, viruses, viroids, mycoplasma, or other microorganisms. In some embodiments, the sample nucleic acid comprises or consists of genomic DNA, subgenomic DNA, chromosomal DNA (e.g., from an isolated chromosome or a portion of a chromosome, e.g., from one or more genes or loci from a chromosome), mitochondrial DNA, chloroplast DNA, plasmid or other episomal-derived DNA (or recombinant DNA contained therein), or double-stranded cDNA made by reverse transcription of RNA using an RNA-dependent DNA polymerase or reverse transcriptase to generate first-strand cDNA and then extending a primer annealed to the first-strand cDNA to generate dsDNA. In some embodiments, the sample nucleic acid comprises multiple dsDNA molecules in or prepared from nucleic acid molecules (e.g., multiple dsDNA molecules in or prepared from genomic DNA or cDNA prepared from RNA in or from a biological (e.g., cell, tissue, organ, organism) or environmental (e.g., water, air, soil, saliva, sputum, urine, feces) source. In some embodiments, the sample nucleic acid is from an in vitro source. For example, in some embodiments, the sample nucleic acid comprises or consists of dsDNA that is prepared in vitro from single-stranded DNA (ssDNA) or from single-stranded or double-stranded RNA (e.g., using methods that are well-known in the art, such as primer extension using a suitable DNA-dependent and/or RNA-dependent DNA polymerase (reverse transcriptase). In some embodiments, the sample nucleic acid comprises or consists of dsDNA that is prepared from all or a portion of one or more double-stranded or single-stranded DNA or RNA molecules using any methods known in the art, including methods for: DNA or RNA amplification (e.g., PCR or reverse-transcriptase-PCR (RT-PCR), transcription-mediated amplification methods, with amplification of all or a portion of one or more nucleic acid molecules); molecular cloning of all or a portion of one or more nucleic acid molecules in a plasmid, fosmid, BAC or other vector that subsequently is replicated in a suitable host cell; or capture of one or more nucleic acid molecules by hybridization, such as by hybridization to DNA probes on an array or microarray.

The disclosed nucleic acid techniques may be implemented as part of a sequencing workflow. The sequencing technique may include incorporating sequencing-by-synthesis methods described in U.S. Patent Publication Nos. 2007/0166705; 2006/0188901; 2006/0240439; 2006/0281109; 2005/0100900; U.S. Pat. No. 7,057,026; WO 05/065814; WO 06/064199; WO 07/010,251, the disclosures of which are incorporated herein by reference in their entireties. Some embodiments can utilize nanopore sequencing, whereby sample nucleic acid strands, or nucleotides exonucleolytically removed from sample nucleic acids, pass through a nanopore. As the sample nucleic acids or nucleotides pass through the nanopore, each type of base can be identified by measuring fluctuations in the electrical conductance of the pore (U.S. Patent No. 7,001,792; Soni & Meller, Clin. Chem. 53, 1996-2001 (2007); Healy, Nanomed. 2, 459-481 (2007); and Cockroft, et al. J. Am. Chem. Soc. 130, 818-820

(2008), the disclosures of which are incorporated herein by reference in their entireties). Yet other embodiments include detection of a proton released upon incorporation of a nucleotide into an extension product. For example, sequencing based on detection of released protons can use an electrical detector and associated techniques that are commercially available from Ion Torrent (Guilford, CT, a Life Technologies subsidiary) or sequencing methods and systems described in US 2009/0026082 A1; US 2009/0127589 A1; US 2010/0137143 A1; or US 2010/0282617 A1, each of which is incorporated herein by reference in its entirety. Particular embodiments can utilize methods involving the real-time monitoring of DNA polymerase activity. Nucleotide incorporations can be detected through fluorescence resonance energy transfer (FRET) interactions between a fluorophore-bearing polymerase and γ-phosphate-labeled nucleotides, or with zeromode waveguides as described, for example, in Levene et al. Science 299, 682-686 (2003); Lundquist et al. Opt. Lett. 33, 1026-1028 (2008); Korlach et al. Proc. Natl. Acad. Sci. USA 105, 1176-1181 (2008), the disclosures of which are incorporated herein by reference in their entireties. Other suitable alternative techniques include, for example, fluorescent in situ sequencing (FISSEQ), and Massively Parallel Signature Sequencing (MPSS). In particular embodiments, the sequencing device 260 may be an iSeq from Illumina (La Jolla, CA).

In some embodiments, the modified nucleotides 12 may include a cleavable moiety that is subject to photochemical cleavage to permit liberation of a 3' end of the oligonucleotide adapter. The cleavage at the cleave site breaks a link of the oligonucleotide adapter 24 to a linker 20, e.g., a carbon linker. Photochemical cleavage encompasses any method which utilizes light energy in order to achieve cleavage of nucleic acids, for example, one or both strands of a double-stranded nucleic acid molecule. A site for photochemical cleavage can be provided by a non-nucleotide chemical moiety in a nucleic acid, such as phosphoramidite [4-(4,4'-dimethoxytrityloxy)butyramidomethyl)-1-(2-nitrophenyl)-ethyl]-2-cyanoethyl-(N,N-diisopropyl)-phosphoramidite) (Glen Research, Sterling, Va., USA, Cat No. 10-4913-XX).

In some embodiments, the oligonucleotide adapters 23 can include an affinity binder or an affinity tag that functions as a handle to permit pulldown or purification of nucleic acids that have incorporated modified nucleotides 12. Affinity tags can be useful for the bulk separation of target nucleic acids. As used herein, the term "affinity binder" can refer to a component of a multi-component complex, wherein the components of the multi-component complex specifically interact with or bind to each other. For example an affinity tag can include biotin or His that can bind streptavidin or nickel, respectively. Other examples of multiple-component affinity tag complexes include, ligands and their receptors, for example, avidin-biotin, streptavidin-biotin, and derivatives of biotin, streptavidin, or avidin, including, but not limited to, 2-iminobiotin, desthiobiotin, NeutrAvidin (Molecular Probes, Eugene, Oreg.), CaptAvidin (Molecular Probes), and the like; binding proteins/peptides, including maltose-maltose binding protein (MBP), calcium-calcium binding protein/peptide (CBP); antigen-antibody, including epitope tags, and their corresponding anti-epitope antibodies; haptens, for example, dinitrophenyl and digoxigenin, and their corresponding antibodies; aptamers and their corresponding targets; poly-His tags (e.g., penta-His and hexa-His) and their binding partners including corresponding immobilized metal ion affinity chromatography (IMAC) materials and anti-poly-His antibodies; fluorophores and anti-fluorophore antibodies; and the like.

The disclosed modified nucleotides 12 (e.g., oligo-modified nucleotide analogues, oNTPS) are non-naturally occurring molecules. In embodiments, the modified nucleotides are incorporated into naturally-occurring nucleic acid sequences and/or synthetic nucleic acid sequences. In embodiments, the modified nucleotides are coupled to oligonucleotide adapters that include non-naturally occurring nucleic acid sequences, such as universal adapter sequences suitable for sequencing or other nucleic acid manipulation workflows. For multiplexed reactions, different modified nucleotides 12 may be used for respective different samples that differ by distinguishable sample index sequences of the oligonucleotide adapters but that are otherwise the same.

Embodiments of the disclosure include compositions of modified nucleotides 12 and/or nucleic acids having one or more incorporated modified nucleotides 12. Embodiments of the disclosure include nucleic acids generated as part of sequencing library preparation that have 3' adapters added via incorporation of the modified nucleotide 12 and base-to-backbone swap of the adapter to the 3' end of the incorporated modified nucleotide 12 via ligation and cleavage at a linker extending from the nucleobase to liberate a 3' end of the adapter. Embodiments of the disclosure include sample preparation kits that include modified nucleotides 12 as well as relevant reagents for the workflow, e.g., a sequencing library preparation. Relevant reagents may include chemical reagents or enzymes such as ligases and/or polymerases as discussed herein.

This written description uses examples as part of the disclosure to enable any person skilled in the art to practice the disclosed embodiments, including making and using any devices or systems and performing any incorporated methods. The patentable scope is defined by the claims, and may include other examples that occur to those skilled in the art. Such other examples are intended to be within the scope of the claims if they have structural elements that do not differ from the literal language of the claims, or if they include equivalent structural elements with insubstantial differences from the literal languages of the claims. Aspects and embodiments of the invention will now be described by reference to the following numbered clauses.

Aspects and embodiments of the invention are described below by reference to the following numbered clauses.
Clause 1: An oligo-modified nucleic acid analogue composition, comprising: a modified nucleotide comprising: a ribose; a 5' phosphate group coupled to the ribose; a 3' reactive group coupled to the ribose; and an oligonucleotide adapter coupled to the ribose by a linker and terminating in a 5' oligonucleotide end.
Clause 2: The composition of clause 1, wherein the oligonucleotide adapter is coupled to a 1' position of the ribose via the linker.
Clause 3: The composition of clause 1, wherein the modified nucleotide comprises a nucleobase coupled to a 1' position of the ribose, wherein the linker extends from the nucleobase.
Clause 4: The composition of clause 3, wherein the nucleobase is uracil, thymine, cytosine, adenine, or guanine.
Clause 5: The composition of clause 3, wherein the composition comprises a plurality of modified nucleotides comprising a mix of nucleotide bases.
Clause 6: The composition of clause 5, comprising a plurality of unmodified nucleotides, the unmodified nucleotides comprising one or more of uracil, thymine, cytosine, adenine, or guanine.
Clause 7: The composition of clause 1, wherein the linker comprises a carbon chain comprising two or more carbons, and wherein the oligonucleotide adapter is coupled directly or indirectly to the carbon chain.
Clause 8: The composition of clause 1, wherein the linker is a cleavable linker.
Clause 9: The composition of clause 8, wherein the cleavable linker comprises an enzymatically cleavable, chemically cleavable, or photocleavable molecule.
Clause 10: The composition of clause 1, wherein the 5' oligonucleotide end is reactive with the 3' reactive group to couple the 5' oligonucleotide end to the ribose at a 3' position.
Clause 11: The composition of clause 1, comprising a reversible blocker on the 5' oligonucleotide end or the 3' reactive group.
Clause 12: The composition of clause 1, wherein the 5' oligonucleotide end comprises a phosphate group or an alkyne group.
Clause 13: The composition of clause 1, wherein the 3' reactive group comprises a hydroxyl group or an azide.
Clause 14: The composition of clause 1, wherein the oligonucleotide adapter comprises a primer binding site, a capture site, an index, or a combination thereof.
Clause 15: The composition of clause 1, wherein the oligonucleotide adapter is coupled to an affinity binder.
Clause 16: The composition of clause 1, wherein the oligonucleotide adapter is single-stranded.
Clause 17: The composition of clause 1, wherein the oligonucleotide adapter comprises a sequence hybridized to a recognition sequence extending from the linker.
Clause 18: The composition of clause 1, wherein the oligonucleotide adapter comprises a forked adapter.
Clause 19. The composition of clause 1, wherein the oligonucleotide adapter is 10 to 1000 nucleotides in length.
Clause 20. The composition of clause 1, wherein the ribose is a deoxyribose or a dideoxyribose.
Clause 21: An oligo-modified nucleic acid analogue composition, comprising:
   a modified nucleotide comprising:
   a ribose;
   a 5' phosphate group coupled to the ribose;
   a 3' reactive group coupled to the ribose; and
   an oligonucleotide adapter coupled to the ribose by a linker and terminating in a 3' oligonucleotide end.
Clause 22: A method of modifying a nucleic acid, comprising:
   providing a nucleic acid;
   contacting the nucleic acid with a modified nucleotide comprising:
      a deoxyribose;
      a 5' phosphate group coupled to the deoxyribose;
      a 3' reactive group coupled to the deoxyribose; and
      an oligonucleotide adapter coupled to the deoxyribose by a linker and terminating in a 5' oligonucleotide end;
   incorporating the modified nucleotide onto a 3' end of the nucleic acid via the 5' phosphate group to generate an extended nucleic acid; and
   reacting the 5' oligonucleotide end of the oligonucleotide adapter on the extended nucleic acid with the 3' reactive group to couple the 5' oligonucleotide end to the deoxyribose and such that the oligonucleotide adapter forms a loop.
Clause 23: The method of clause 22, further comprising cleaving the linker to liberate a 3' end of the oligonucleotide adapter.
Clause 24: The method of clause 23, wherein the cleaving comprises enzymatic cleaving, chemical cleaving, or photocleaving.
Clause 25: The method of clause 23, wherein the cleaving comprises cleaving a uracil of the linker to leave an abasic site using a uracil glycosylase.
Clause 26: The method of clause 22, wherein the incorporating comprises using a polymerase.
Clause 27: The method of clause 26, wherein the polymerase does not have 5' to 3' exonuclease activity.
Clause 28: The method of clause 22, wherein the nucleic acid is a double-stranded nucleic acid, and wherein a 5' end of at least one strand of the double-stranded nucleic acid comprises an adapter.
Clause 29: The method of clause 28, wherein the adapter is a forked adapter.
Clause 30: The method of clause 28, comprising tagmenting a nucleic acid sample to provide the double-stranded nucleic acid, wherein the tagmenting couples a 5' adapter to 5' ends of the double-stranded nucleic acid.
Clause 31: The method of clause 22, wherein the 3' end is a 3' end of a primer annealed to a template.
Clause 32: The method of clause 22, comprising deblocking the 3' reactive group and/or the 5' oligonucleotide end before the reacting.
Clause 33: The method of clause 22, comprising:
   cleaving the linker to liberate a 3' end of the oligonucleotide adapter;
   forming a second loop with the oligonucleotide adapter, the second loop comprising the liberated 3' end of the oligonucleotide adapter; and
   extending from the liberated 3' end to synthesize a copy of the nucleic acid.
Clause 34: A method of modifying a nucleic acid, comprising:
   contacting a single-stranded nucleic acid with a modified nucleotide comprising:
      a deoxyribose;
      a 5' phosphate group coupled to the deoxyribose; and
      a single-stranded oligonucleotide adapter coupled to the deoxyribose and terminating in a 5' oligonucleotide end;
   using a polymerase to incorporate the modified nucleotide onto a 3' end of the single-stranded nucleic acid via the 5' phosphate group to generate an extended single-stranded nucleic acid;
   annealing a primer comprising a recognition site for a 5' region of the single-stranded oligonucleotide adapter; and
   extending the primer to synthesize a complementary strand of the single-stranded nucleic acid.
Clause 35: A method of preparing a sequencing library, comprising:
   providing a double-stranded nucleic acid sample;
   tagmenting the double-stranded nucleic acid sample using transposome homodimers to incorporate a first adapter on 5' ends of double-stranded fragments generated from the double-stranded nucleic acid sample;
   contacting the double-stranded fragments with modified nucleotides comprising:
      a deoxyribose;
      a 3' reactive group coupled to the deoxyribose; and
      an oligonucleotide adapter coupled to the deoxyribose by a cleavable linker and terminating in a 5' oligonucleotide end;
   incorporating the modified nucleotides onto 3' ends of the double-stranded fragments to generate an extended nucleic acid;
   reacting respective 5' oligonucleotide ends of the modified nucleotides with corresponding 3' reactive groups to couple the 5' oligonucleotide ends to the deoxyribose and such that oligonucleotide adapters of the modified nucleotides form loops; and
   cleaving cleavable linkers of the modified nucleotides liberate 3' ends of the oligonucleotide adapter to generate adapterized double-stranded nucleic fragments of a sequencing library.
Clause 36: The method of clause 35, comprising sequencing the sequencing library.
Clause 37: The method of clause 35, wherein the oligonucleotide adapters all have a same sequence.
Clause 38: A method of generating a sequencing library, comprising:
   providing a double-stranded nucleic acid;
   contacting the double-stranded nucleic acid with modified adenosines, an individual modified adenosine comprising:
      a deoxyribose;
      a 5' phosphate group coupled to the deoxyribose;
      a 3' reactive group coupled to the deoxyribose;
      an adenine nucleobase coupled to the deoxyribose; and
      an oligonucleotide adapter coupled to the deoxyribose or the adenine nucleobase by a linker, wherein the oligonucleotide adapter comprises a forked adapter comprising a first fork, a second fork, and double-stranded portion, the double-stranded portion comprising a 3' thymine overhang, and wherein the linker is coupled to the first fork;
   incorporating the modified adenosines onto 3' ends of the double-stranded nucleic acid via the 5' phosphate group to generate an extended nucleic acid comprising 3' modified adenosine ends;
   ligating the double-stranded portion of the forked adapter to the 3' modified adenosine ends of the double-stranded nucleic acid via the 3' thymine overhang; and
   cleaving the linker from the first fork subsequent to the ligating to generate double-stranded nucleic acid having the forked adapter at both ends.
Clause 39: The method of clause 38, wherein the double-stranded nucleic acid is a blunt-ended nucleic acid fragment and wherein incorporating the modified adenosines comprises creating an adenosine tail.
Clause 40: The method of clause 38, wherein the forked adapter comprises a primer binding sequence.
Clause 41: The method of clause 38, wherein the 3' thymine overhang is on a same strand as the first fork.
Clause 42: A method of generating a sequencing library, comprising:
   providing a double-stranded nucleic acid;
   contacting the double-stranded nucleic acid with modified adenosines, an individual modified adenosine comprising:
      a deoxyribose;
      a 5' phosphate group coupled to the deoxyribose;
      a 3' reactive group coupled to the deoxyribose;
      an adenine nucleobase coupled to the deoxyribose; and
      a first oligonucleotide adapter coupled to the deoxyribose or the adenine nucleobase by a first linker;
   incorporating the modified adenosines onto 3' ends of the double-stranded nucleic acid via the 5' phosphate group to generate an extended nucleic acid comprising 3' modified adenosine ends;
   contacting the extended nucleic acid comprising the 3' modified adenosine ends with a forked adapter comprising a first fork, a second fork, a double-stranded portion, the double-stranded portion comprising a 3' thymine overhang, and a second oligonucleotide adapter complementary to the first oligonucleotide adapter, the second oligonucleotide adapter extending from the first fork or the second fork via a second linker, to allow the first oligonucleotide adapter to hybridize to the second oligonucleotide adapter;
   ligating the double-stranded portion of the forked adapter to the 3' modified adenosine ends of the double-stranded nucleic acid via the 3' thymine overhang; and
   cleaving the first linker and the second linker to generate double-stranded nucleic acid having the forked adapter at both ends.
Clause 43: The method of clause 42, wherein the ligating occurs subsequent to the hybridization of the first oligonucleotide adapter to the second oligonucleotide adapter.
Clause 44: The method of clause 42, wherein the first oligonucleotide adapter and the second oligonucleotide adapter have a same self-complementary sequence.
Clause 45: The method of clause 42, wherein the 3' thymine overhang is on a different strand than the second oligonucleotide adapter.
Clause 46: A nucleic acid fragment comprising:
   a single or double-stranded nucleic acid fragment; and
   a modified nucleotide coupled to a 3' end of the nucleic acid fragment, the modified nucleotide comprising: an oligonucleotide adapter coupled to a ribose by a linker at a first end and terminating in a 5' or 3' oligonucleotide end at a second end.
Clause 47: The nucleic acid fragment of clause 46, wherein the oligonucleotide adapter is coupled to a 1' position of the ribose via the linker.
Clause 48: The nucleic acid fragment of clause 46, wherein the modified nucleotide comprises a nucleobase coupled to a 1' position of the ribose, wherein the linker extends from the nucleobase.
Clause 49: The nucleic acid fragment of clause 48, wherein the nucleotide base is uracil, thymine, cytosine, adenine, or guanine.
Clause 50: The nucleic acid fragment of clause 46, wherein the oligonucleotide adapter is single-stranded.
Clause 51: The nucleic acid fragment of clause 46, wherein the oligonucleotide adapter is hybridized to a tail of a forked adapter.
Clause 52: The nucleic acid fragment of clause 46, wherein the nucleic acid fragment is a partially single-stranded RNA.
Clause 53: The nucleic acid fragment of clause 46, wherein the nucleic acid fragment is a double-stranded DNA.
Clause 54: The nucleic acid fragment of clause 53, wherein the modified nucleotide is incorporated at a 3' recessed end of the double-stranded DNA.
Clause 55: The nucleic acid fragment of clause 53, wherein the modified nucleotide is incorporated at a 3' blunt end of the double-stranded DNA.
Clause 56: A method of modifying a nucleic acid, comprising:
   contacting a double-stranded nucleic acid with an a modified nucleotide comprising:
      a deoxyribose;
      a 5' phosphate group coupled to the deoxyribose; and
      a single-stranded oligonucleotide adapter coupled to the deoxyribose via a linker at a first end and terminating in a 3' oligonucleotide end at a second end;
   incorporating the modified nucleotide onto a 3' end of a first strand of the double-stranded nucleic acid via the 5' phosphate group to generate an extended first strand;
   annealing a primer comprising a recognition site for a 3' region of the single-stranded oligonucleotide adapter; and
   extending the primer using a polymerase with 5' to 3' exonuclease activity to synthesize a complementary strand of the single-stranded oligonucleotide adapter while degrading a 5' portion of a second strand of the double-stranded nucleic acid.
Clause 57: The method of clause 56, comprising ligating a 3' end of the complementary strand to a 5' end of an undegraded portion of the second strand.
Clause 58: A method of modifying a nucleic acid, comprising:
   contacting a double-stranded nucleic acid with an a modified nucleotide comprising:
   a deoxyribose;
   a 5' phosphate group coupled to the deoxyribose; and
   a single-stranded oligonucleotide adapter coupled to the deoxyribose;
   incorporating the modified nucleotide onto a recessed 3' end of a first strand of the double-stranded nucleic acid via the 5' phosphate group to generate an extended first strand;
   extending the first strand from a 3' end of the modified nucleotide;
   annealing a single-stranded portion of a forked adapter to the single-stranded oligonucleotide adapter; and
   ligating a double-stranded portion of the forked adapter to ends of the double-stranded nucleic acid.
Clause 59: The method of clause 56, wherein the first end is extended to form a blunt end to which the double-stranded portion is ligated.
Clause 60: The method of clause 56, wherein the single-stranded oligonucleotide adapter terminates in a free 3' oligonucleotide end.
Clause 61: A method of modifying a nucleic acid, comprising:
   contacting a single-stranded RNA with a plurality of single-stranded oligonucleotides comprising a 3' random portion and a 5' fixed sequence portion such that a 3' random portion of one of the plurality of single-stranded oligonucleotides anneals to a 3' end of the single-stranded RNA and such that the 5' fixed sequence portion does not anneal to the single-stranded RNA; and
   incorporating a modified nucleotide onto a 3' end of the single-stranded RNA using the fixed sequence portion as a template, wherein the modified nucleotide comprises:
      a deoxyribose;
      a 5' phosphate group coupled to the deoxyribose; and
      a single-stranded oligonucleotide adapter coupled to the deoxyribose and terminating in a free 3' end.

## Claims

1. An oligo-modified nucleic acid analogue composition, comprising:
a modified nucleotide comprising:
a ribose;
a 5' phosphate group coupled to the ribose;
a 3' reactive group coupled to the ribose; and
an oligonucleotide adapter coupled to the ribose by a linker and terminating in a 3' oligonucleotide end.

2. The composition of claim 1, wherein the oligonucleotide adapter is coupled to 3' position of the ribose via the linker.

3. The composition of claim 1, wherein the oligonucleotide adapter is single-stranded.

4. The composition of claim 1, wherein the oligonucleotide adapter is coupled to a 1' position of the ribose via the linker.

5. The composition of claim 1, wherein the 3' reactive group comprises a hydroxyl group or an azide.

6. The composition of claim 1, wherein the modified nucleotide comprises a nucleobase coupled to a 1' position of the ribose.

7. The composition of claim 1, wherein the oligonucleotide adapter is coupled to an affinity binder.

8. A nucleic acid fragment comprising:
a single or double-stranded nucleic acid fragment; and
a modified nucleotide coupled to a 3' end of the nucleic acid fragment, the modified nucleotide comprising: an oligonucleotide adapter coupled to a ribose by a linker at a first end and terminating in a 5' or 3' oligonucleotide end at a second end.

9. The nucleic acid fragment of claim 8, wherein the oligonucleotide adapter is coupled to a 1' position of the ribose via the linker.

10. The nucleic acid fragment of claim 8, wherein the modified nucleotide comprises a nucleobase coupled to a 1' position of the ribose, wherein the linker extends from the nucleobase.

11. The nucleic acid fragment of claim 10, wherein the nucleobase is uracil, thymine, cytosine, adenine, or guanine.

12. The nucleic acid fragment of claim 8, wherein the oligonucleotide adapter is hybridized to a tail of a forked adapter.

13. The nucleic acid fragment of claim 8, wherein the nucleic acid fragment is a double-stranded DNA.

14. The nucleic acid fragment of claim 13, wherein the modified nucleotide is incorporated at a 3' recessed end of the double-stranded DNA.

15. The nucleic acid fragment of claim 13, wherein the modified nucleotide is incorporated at a 3' blunt end of the double-stranded DNA.
